# EUROPEAN PATENT SPECIFICATION

(11) **EP 3 830 284 B1**
(45) Date of publication and mention of the grant of the patent: **19.02.2025**
(21) Application number: 19748918.0
(22) Date of filing: 26.07.2019
(51) Int. Cl.: C12Q 1/34, C12Q 1/689, G01N 33/50, G01N 33/569, G01N 33/68

(54) **BACTERIAL VAGINOSIS DIAGNOSTIC**
BAKTERIELLE VAGINOSE-DIAGNOSE
DIAGNOSTIC DE VAGINOSE BACTÉRIENNE

(30) Priority: 27.07.2018 GB 201812331
(43) Date of publication of application: 09.06.2021
(73) Proprietor: MOLOGIC LTD., Thurleigh, Bedfordshire MK44 2YP (GB)
(72) Inventor: DAVIS, Paul, Thurleigh, Bedfordshire MK44 2YP (GB); SCHOUTEN, James, Thurleigh, Bedfordshire MK44 2YP (GB)
(74) Representative: Boult Wade Tennant LLP
(86) International application number: PCT/GB2019/052101
(87) International publication number: WO 2020/021281

(56) References cited:
- WO-A2-2006/010143
- US-A1- 2005 096 263
- NAKANO V ET AL: "A rapid assay of the sialidase activity in species of the Bacteroides fragilis group by using peanut lectin hemagglutination", ANAEROBE, LONDON, GB, vol. 12, no. 5-6, 1 October 2006 (2006-10-01), pages 238 - 241, XP024917600, ISSN: 1075-9964, [retrieved on 20061001], DOI: 10.1016/J.ANAEROBE.2006.07.003
- GWLADYS POURCEAU ET AL: "Measurement of Enzymatic Activity and Specificity of Human and Avian Influenza Neuraminidases from Whole Virus by Glycoarray and MALDI-TOF Mass Spectrometry", CHEMBIOCHEM, vol. 12, no. 13, 7 July 2011 (2011-07-07), pages 2071 - 2080, XP055635667, ISSN: 1439-4227, DOI: 10.1002/cbic.201100128
- TOMIN A ET AL: "Novel assay for direct fluorescent imaging of sialidase activity", CLINICAL AND BIOMEDICAL SPECTROSCOPY AND IMAGING II, SPIE, 1000 20TH ST. BELLINGHAM WA 98225-6705 USA, vol. 8087, no. 1, 9 June 2011 (2011-06-09), pages 1 - 8, XP060015337, DOI: 10.1117/12.889172

## Description

### FIELD OF THE INVENTION

The present invention relates to detecting cleavage activity of a sialidase enzyme and the use thereof in detecting bacterial vaginosis. In particular, the invention provides specifically-designed peptides and indicator molecules useful for detecting cleavage activity of a sialidase enzyme. Various other aspects of the invention include an enzyme detection device, kit, method and use for detecting or measuring the presence in a test sample of the activity of a sialidase enzyme capable of cleaving an indicator molecule of the invention.

### BACKGROUND TO THE INVENTION

Sialidase enzymes (otherwise known as neuraminidases) are glycoside hydrolase enzymes split into two main classes that cleave exo or endo (poly-)sialic acids (EC 3.2.1.18 and EC 3.2.1.129 respectively). Sialic acids are N- or O-substituted derivatives of neuraminic acid (shown below):

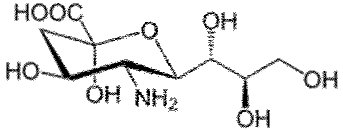

Sialic acids are generally found in nature in glycoproteins and glycolipids (in particular gangliosides). Viral, bacterial and mammalian sialidases are all known in nature. Elevated levels of bacterial sialidase activity are known to act as a diagnostic marker for bacterial vaginosis (BV; Briselden et. al., J. Clin. Microbiol., 1992, vol. 30, pages 663-666). The theory behind this is based on the bacterial imbalance that can occur in the vagina and the transition from a typical healthy environment high in lactobacilli to an anaerobic driven microbiome (Petrova et. al., Front. Physiol., 2015, 6:81). Certain anaerobes excrete sialidase enzyme and compromise the natural mucus barriers in the vagina. Nonetheless, the exact mechanism for BV is still to be fully established. Patients suffering from symptomatic BV can experience significant discomfort, vaginal discharge, an unpleasant odour and more generally a feeling of lack of control over their vaginal health (Bilardi et. al., Plos One, 2013, vol. 8, e74378). Treatment pathways typically involve topical or oral antibiotics. Topical prebiotic treatments (e.g.: VH essentials ^{®}), and pH gel (e.g. balance active BV) are also available over the counter. BV can be intractable and patients can suffer a relapse after antibiotic treatment (Bilardi et. al., Plos One, 2013, vol. 8, e74378).

Some of the intractable cases are believed to be linked to the formation of biofilms in the vagina (Verstraelen & Swidsinski, Curr. Opin. Infect. Dis., 2013, 26:86-89). The risks in not picking up the presence of BV are a greater susceptibility to sexually transmitted infection (including HIV) and an associated risk of pre-term birth. The pre-term birth risk is believed to be linked to premature weakening of the cervical mucus barrier (Lewis et. al., J. Biol. Chem., 2013, vol. 288, pages 12067-12079). Currently in the clinic, the test for BV is based on a vaginal sample smear, sent for analysis in the lab. Methods of analysis include looking for clue cells, the potassium hydroxide (KOH) whiff test, or applying set criteria such as the Nugent score (Nash, Jungmann, & Gubert, BMJ Learning, 2015, 1-29). Tomin et al (Med. Spec. and Imaging II, 8087) discloses a sialidase activity assay based on the fluorescent substrate 4-MU-NA (2'-(4-Methylumbelliferyl)-alpha-D-N-acetylneuraminic acid). The use of a rapid test at point of care would benefit the patient by delivering a quick answer, putting them on an immediate treatment plan and speed up the overall process for delivering outcomes. Likewise there would be potential benefit to the healthcare provider, by avoiding outsourcing to a lab and the resource and time costs involved and keeping the patient time down to a single session in the first instance.

Two main types of products for diagnosing BV are currently available in the USA and Europe. One set of products is based on a colour changing swab that measures pH. The other set of products is also colourimetric and based on sialidase activity measurement. The pH-based products are available at point of care and over the counter and include VS-SENSE PRO^{®} (sold by Common Sense) and Canestest^{®} (sold by Canesten). The main product currently on sale based on sialidase detection is BV Blue (sold by Sekisui Diagnostics and Gryphus Diagnostics). BV Blue is a swab-based test; a sample swab is placed in an indicator solution which will change colour to indicate the presence of sialidase activity in the sample. The test takes approximately 15 minutes to run.

The pH-based tests lack accuracy since various conditions can cause a change in the local pH of the vagina (e.g. Candidiasis and Trichonomiasis as well as BV). The known tests such as BV Blue which detect sialidase activity lack sensitivity.

### DESCRIPTION OF THE INVENTION

The present invention results from attempts to improve sensitivity and/or specificity of sialidase activity detection. The present inventors have now specifically designed peptides and corresponding indicator molecules useful for detecting cleavage activity of a sialidase enzyme. As described further herein, each peptide is conjugated to a sialyl group via a galactosyl group (and thus functions as a substrate for a sialidase enzyme) wherein the peptide architecture is specifically designed so that, once the sialyl group has been cleaved from the peptide by one or more sialidase enzymes present in the sample, the de-sialylated derivative of the peptide is recognised and bound by specific binding molecules (e.g. antibodies). In preferred embodiments, the peptide incorporates one or more amino acids which enhance the peptide's resistance to protease cleavage.

The appended claims define aspects and embodiments of the invention.

Accordingly, in one aspect, the invention provides a peptide as defined in claim 9. In another aspect, the invention provides an indicator molecule as defined in claim 8. The provided peptides and indicator molecules are based on a peptide of formula I, which is described herein. Thus, described herein is a peptide comprising a sequence according to the following formula:

X₁-X₂-X₃[Gal-Sial]-X₄-X₅ (Formula I) (SEQ ID NO: 9)

wherein:
(i) Sial is a sialyl group;
(ii) X₃ is a natural or non-natural amino acid comprising a glycosyl acceptor group; and
(iii) X₁, X₂, X₄ and X₅ are independently selected from any amino acid provided that at least one of X₁, X₂, X₄ and X₅ is a _{D}-amino acid and/or a non-standard amino acid or a non-natural amino acid.

According to all aspects of the invention, by "sialyl group" is meant a sialic acid substituent wherein a sialic acid is an *N*- or *O*-substituted derivative of neuraminic acid (e.g. *N-*acetylneuraminic acid; termed 'Neu5Ac' or 'NANA').

According to all aspects of the invention, by "Gal" is meant a galactosyl substituent. In preferred embodiments, the galactosyl substituent is a radical of the following structure:

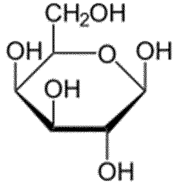

In particular embodiments, the galactosyl subtituent is a radical of β-galactose. In such embodiments, the galactosyl substituent is *O*-linked to the sialyl group. All possible regioisomers are encompassed by the invention. The skilled person will also appreciate that other saccharides may be used in place of a galactosyl group, such as radicals of glucose, fructose, lactose, maltose and sucrose for example. Thus, reference to a "galactosyl substituent" herein is to be interpreted accordingly.

The -Gal-Sial group is conjugated to X₃ as shown above wherein X₃ is a natural or non-natural amino acid comprising a glycosyl acceptor group. That is to say the side-chain of the amino acid represented by X₃ comprises a nucleophilic substituent that forms a bond with the Gal substituent (i.e. a glycosyl donor). For instance, the nucleophilic substituent may be a hydroxyl or amine substituent. In all embodiments, X₃ is selected from serine (Ser), threonine (Thr), tyrosine (Tyr), hydroxylysine (Hyl), hydroxyproline (Hyp), asparagine (Asn), arginine (Arg) and phosphoserine (SEP). In preferred embodiments, X₃ is Ser.

It should be understood from Formula I that the -Gal-Sial group is bonded to X₃ only (as indicated by the square brackets in Formula I). It does not bridge X₃ to X₄. For the avoidance of doubt, an alternative and equivalent formula (la) is shown below:

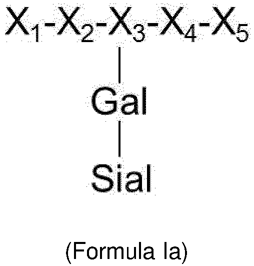

Hence, the -Gal-Sial group is branched from the peptide backbone. Thus, the -Gal substituent is positioned centrally in the structure so that it is flanked by amino acids X₁, X₂, X₄ and X₅. Consequently, as described further herein, binding molecules (e.g. antibodies) can be generated with very high affinity and specificity for the de-sialylated derivative of each peptide and the generation of peripheral binding molecules (e.g. antibodies) which lack interaction with the Gal substituent is minimised.

X₁, X₂, X₄ and X₅ are independently selected from any amino acid (natural and non-natural) provided that at least one of X₁, X₂, X₄ and X₅ is a _{D}-amino acid and/or (i) a non-standard amino acid or (ii) a non-natural amino acid. Non-standard and non-natural amino acids add to the sequence diversity and help promote an immune response in a host organism when generating binding molecules which are antibodies with high affinity for the de-sialylated derivative of the peptide. _{D}-amino acids help to reduce susceptibility to proteases. In particular embodiments, at least two, three or all four of X₁, X₂, X₄ and X₅ are a _{D}-amino acid and/or a non-standard amino acid or a non-natural amino acid.

The skilled person will be very familiar with the terms "natural" and "non-natural" amino acids. For the avoidance of doubt, a "natural" amino acid is one found in nature and includes both standard and non-standard amino acids. As known in the art, standard amino acids are those that are encoded directly by triplet codons in the universal genetic code. Conversely, non-standard amino acids are those which are found in nature but which are not encoded directly by triplet codons in the universal genetic code. "Non-natural" amino acids are those not found in nature but which only exist via artificial synthesis. As used herein, reference to an amino acid by name with no "_{D}" or "_{L}" prefix is used to mean both D and L stereoisomers. Use of a "_{D}" or "_{L}" prefix denotes that specific stereoisomer.

The "de-sialylated derivative" of a peptide or indicator molecule is the product produced by cleavage of a sialyl group from the peptide or indicator molecule, typically by a sialidase enzyme. Thus, the "de-sialylated derivative" of a peptide or indicator molecule typically differs from the sialylated peptide or indicator molecule only, or substantially only, with regard to the absence of the sialyl group. Any reference herein to a "de-sialylated derivative" of a peptide or indicator molecule should therefore be understood to mean the de-sialylated form of that peptide or indicator molecule.

Further increasing the diversity of the peptide sequence topology is beneficial as this has been shown to further promote the generation of binding molecules with very high affinity and specificity for the de-sialylated derivative of each peptide. This is particularly relevant for binding molecules which are antibodies. Thus, in preferred embodiments, X₁, X₂, X₄ and X₅ comprise at least two, three or four different amino acids. In some embodiments, at least one of X₁, X₂, X₄ and X₅ is a hydrophobic amino acid. For instance, at least one of X₁, X₂, X₄ and X₅ may be selected from alanine (Ala), valine (Val), isoleucine (Ile), leucine (Leu), methionine (Met), phenylalanine (Phe), tyrosine (Tyr), tryptophan (Trp), proline (Pro), _{D}-alanine (_{D}Ala), 1-aminocyclohexane-carboxylic acid (Cyc), β-alanine (βAla), norleucine (Nle), norvaline (Nva), 2'-(aminomethyl)biphenyl-2-carboxylic acid (Bip) and cyclohexylalanine (Cha). Preferably, the L-stereoisomer of Nle, Nva and Cha is employed. In further embodiments, at least two or three of X₁, X₂, X₄ and X₅ are a hydrophobic amino acid. All four of X₁, X₂, X₄ and X₅ may be a hydrophobic amino acid provided at least one of X₁, X₂, X₄ and X₅ is a _{D}-amino acid and/or a non-standard amino acid or a non-natural amino acid. In particular embodiments, the at least one hydrophobic amino acid is Ala. In further embodiments, X₁ and X₂ are both Ala. In preferred embodiments, Ala is _{D}Ala or βAla. In further embodiments, each hydrophobic amino acid is selected from _{D}Ala, βAla, Ile, Val, Pro, Nle, Nva, Cyc, Cha and Bip. Additionally or alternatively, in some embodiments at least one of X₁, X₂, X₄ and X₅ is a charged amino acid. For instance, at least one of X₁, X₂, X₄ and X₅ may be selected from arginine (Arg), histidine (His), lysine (Lys), aspartic acid (Asp), glutamic acid (Glu), ornithine (Orn) and phosphoserine (SEP). Preferably, the L-stereoisomer of Orn and the D-stereoisomer of Asp is employed. In further embodiments, at least two or three of X₁, X₂, X₄ and X₅ are a charged amino acid. All four of X₁, X₂, X₄ and X₅ may be a charged amino acid provided at least one of X₁, X₂, X₄ and X₅ is a _{D}-amino acid and/or a non-standard amino acid or a non-natural amino acid. In particular embodiments, each charged amino acid is selected from Arg, Glu, _{D}Asp, _{L}Orn and SEP. Additionally or alternatively, in some embodiments at least one of X₁, X₂, X₄ and X₅ is a polar amino acid. For instance, at least one of X₁, X₂, X₄ and X₅ may be selected from serine (Ser), threonine (Thr), tyrosine (Tyr), asparagine (Asn), glutamine (Gln) and cysteine (Cys). Preferably, the D-stereoisomer of Ser is employed. In further embodiments, at least two or three of X₁, X₂, X₄ and X₅ are a polar amino acid. All four of X₁, X₂, X₄ and X₅ may be a polar amino acid provided at least one of X₁, X₂, X₄ and X₅ is a _{D}-amino acid and/or a non-standard amino acid or a non-natural amino acid. In particular embodiments, each polar amino acid is selected from _{L}Ser, _{D}Ser and Thr. In preferred embodiments, to maximise the diversity of the peptide sequence topology, X₁, X₂, X₄ and X₅ are a combination of hydrophobic, charged and polar amino acids. Pro and βAla in particular are useful in the peptides of the invention as these function as "hinge groups" allowing additional degrees of freedom in the overall structural fold thereby further increasing the diversity of the peptide sequence topology.

In preferred embodiments, the peptide has a molecular weight of less than 5000 daltons. This facilitates stimulation of an immune response in the host organism for the generation of antibodies which bind to the de-sialylated derivative of the peptide. Thus, in particular embodiments, the peptide may be between 5-20 amino acids in length, more preferably 9-20 amino acids in length.

Thus, the peptide may comprise a sequence according to the following formula:

X₁-X₂-X₃-X₄-X₅-X₆-X₇-X₈-X₉-X₁₀-X₁₁-X₁₂[Gal-Sial]-X₁₃-X₁₄-X₁₅-X₁₆-X₁₇-X₁₈-X₁₉-X₂₀ (Formula II) (SEQ ID NO: 10)

wherein:
Sial is a sialyl group
X₁ is absent or Thr
X₂ is absent or _{D}Ala
X₃ is absent or Nle
X₄ is absent or Glu
X₅ is absent or _{D}Ala
X₆ is absent or Arg
X₇ is absent or selected from Glu, Arg, Ser, Nva, BAla
X₈ is absent or selected from _{D}Ser, _{D}Ala, SEP, Cyc
X₉ is absent or selected from Nva, BIP, _{D}Ala, βAla, Orn
X₁₀ is selected from Cyc, Ser, Ile, _{D}Ala, _{D}Ser
X₁₁ is selected from _{D}Ala, Pro, Orn, Nle
X₁₂ is selected from Ser, Thr, Tyr, Hyl, Hyp, Asn, Arg or SEP
X₁₃ is selected from _{D}Ala, BlP, BAla
X₁₄ is selected from Arg, _{D}Asp, Nle, Orn, Nva
X₁₅ is absent or selected from Phe, BlP, Ser, Glu, _{D}Ala, _{D}Ser
X₁₆ is absent or selected from _{D}Ser, Glu
X₁₇ is absent or selected from Val, Ser, Thr
X₁₈ is absent or Cha
X₁₉ is absent or _{D}Ser
X₂₀ is absent or Val.

It should be understood from Formula II that the -Gal-Sial group is bonded to X₁₂ only (as indicated by the square brackets in Formula II). It does not bridge X₁₂ to X₁₃. For the avoidance of doubt, an alternative and equivalent formula (IIa) is shown below:

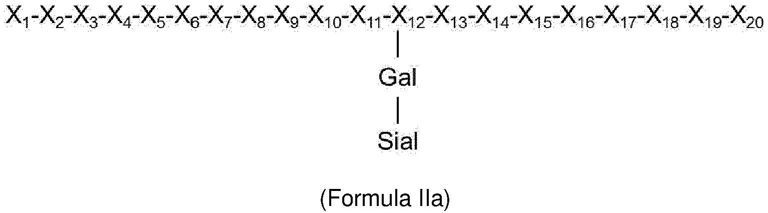

Hence, the -Gal-Sial group is branched from the peptide backbone. Thus, the -Gal substituent is positioned so that it is flanked by amino acids X₁₀₋₁₁ and X₁₃₋₁₄ as well as amino acids X₁₋₉ and X₁₅₋₂₀ (if present). Consequently, as described further herein, binding molecules (e.g. antibodies) can be generated with very high affinity and specificity for the de-sialylated derivative of each peptide and the generation of peripheral binding molecules (e.g. antibodies) which lack interaction with the Gal substituent is minimised.

In preferred embodiments, X₁₂ is Ser.

The invention provides a peptide comprising, or consisting of the following sequence:
(i) Cyc-_{D}Ala-Ser[Gal-Sial]-_{D}Ala-Arg (SEQ ID NO: 11)
(ii) Glu-_{D}Ser-Nva-Cyc-_{D}Ala-Ser[Gal-Sial]-_{D}Ala-Arg-Phe-_{D}Ser-Val (SEQ ID NO: 12)
(iii) Arg-_{D}Ala-Bip-Ser-Pro-Ser[Gal-Sial]-_{D}Ala-_{D}Asp-Ser (SEQ ID NO: 13)
(iv) Ser-Ser(PO₃)-_{D}Ala-Ile-Orn-Ser[Gal-Sial]-_{D}Ala-Nle-Glu (SEQ ID NO: 14)
(v) _{D}Ala-Arg-Nva-_{D}Ser-βAla-_{D}Ala-Nle-Ser[Gal-Sial]-Bip-Orn-_{D}Ala-Glu-Ser (SEQ ID NO: 15); or
(vi) Thr-_{D}Ala-Nle-Glu-_{D}Ala-Arg-ßAla-Cyc-Orn-_{D}Ser-Pro-Ser[Gal-Sial]-ßAla-Nva-_{D}Ser-Glu-Thr-Cha-_{D}Ser-Val (SEQ ID NO: 16);
of which Cyc-_{D}Ala-Ser[Gal-Sial]-_{D}Ala-Arg is particularly preferred.

These specific peptides are been found by the inventors to be particularly useful for detecting sialidase activity and are described further in the Examples section.

Preferably, Nle, Nva, Orn and/or Cha (when present) are _{L}Nle, _{L}Nva, _{L}Orn and _{L}Cha respectively.

In some embodiments, the peptide is biased for cleavage by one or more specific sialidases by constructing the peptide accordingly so that the amino acids flanking the -Gal Sial group ensure specificity and sensitivity of cleavage. Thus, specific sialidase enzymes may have differing affinities for the peptide. This permits the invention to be utilised in order to detect specific sialidase activity in the test sample. As described herein, the peptides of the invention are particularly useful for detecting bacterial vaginosis. Thus, in preferred embodiments, the one or more specific sialidases are of bacterial origin. In particular, the one or more specific sialidases may be from Prevotella, Bacteroides and/or Mobiluncus species and/or *Gardnerella vaginalis.*

In a related aspect, the peptides of the invention are incorporated into an indicator molecule for use in detecting the presence in a test sample of cleavage activity of a sialidase enzyme. The indicator molecule is a core component of the enzyme detection devices, enzyme detection kits, and methods for detecting the presence in a test sample of cleavage activity of a sialidase enzyme described further herein. The indicator molecules described herein have been specifically developed by the inventors in the context of detecting sialidase activity for diagnosing BV based on the inventors' earlier disclosure of the general concept of an indicator molecule for use in detecting cleavage activity of an enzyme and binding molecules which specifically bind to the cleaved product (see PCT/GB2014/053171). Thus, the invention provides an indicator molecule for use in detecting the presence in a test sample of cleavage activity of a sialidase enzyme, the indicator molecule comprising:
(a) a peptide comprising the following sequence:

   X₁-X₂-X₃[Gal-Sial]-X₄-X₅ (SEQ ID NO: 17)

   wherein:
   Sial is a sialyl group;
   X₃ is an amino acid comprising a glycosyl acceptor group and is selected from Ser, Thr, Tyr, Hyl, Hyp, Asn, Arg or phosphoserine (SEP), preferably wherein X₃ is Ser; and
   X₁, X₂, X₄ and X₅ are independently selected from any amino acid provided that at least one, preferably at least two or three, of X₁, X₂, X₄ and X₅ is a _{D}-amino acid and/or a non-standard amino acid or a non-natural amino acid; and
(b) a capture site which remains intact following cleavage of the sialyl group from the indicator molecule by a sialidase enzyme present in the sample.

The capture site is a discrete region of the indicator molecule which mediates binding of the indicator molecule to a capture molecule present within a capture zone (as described in greater detail below). Thus, the capture site is the portion of the indicator molecule responsible for retaining or localising the indicator molecule within the capture zone. Following cleavage of the indicator molecule, the capture site remains intact or substantially intact, such that the site is still recognised and bound by a capture molecule present within the capture zone of the device. Under these circumstances, both intact indicator molecules and the part of the indicator molecules comprising the capture site following cleavage will be bound to capture molecules within the capture zone. The capture site may comprise any suitable molecule, for example a biotin molecule or an oxime moiety. The capture site may be at the N- or C-terminus of the peptide. Key to effectiveness of the indicator molecule is immobilization via the interaction between capture site and a capture molecule at the capture zone and simultaneous binding by a binding molecule after cleavage has occurred.

Thus, the intact indicator molecule may comprise a capture site moiety linked to a peptide of the invention, which peptide comprises a first sialyl group. Cleavage of the indicator molecule by a sialidase enzyme yields a fragment comprising the capture site moiety and the de-sialylated form of the peptide. The fragment (i.e. the cleaved indicator molecule) differs structurally from the intact indicator molecule in that it lacks the first sialyl group that is present in the intact indicator molecule. This lack of the first sialyl group reveals or exposes on the fragment an epitope (a novel binding site) that is absent, hidden or inaccessible in the intact indicator molecule. The intact indicator molecule may therefore be considered to comprise a cryptic epitope. As discussed below, the binding molecules of the devices, kits, and methods of the invention are specific for this epitope and therefore bind only, or preferentially, to the indicator fragment compared to the intact indicator.

The peptide of the indicator molecule and the capture site may be associated by any means known to one of skill in the art. In a preferred embodiment, the peptide and capture site may be associated via a direct covalent linkage. The peptide and capture site may be immediately adjacent or may be separated by a linker or spacer, for example, a polyethylene glycol (PEG) moiety. In some embodiments, the peptide is linked to a biotin group at its N- or C- terminus via a linker comprising, consisting essentially of or consisting of a polyethylene glycol moiety. One or more additional amino acids may be present at the N- or C-terminus of the peptide to link the linker group (such as PEG) to the peptide. The one or more additional amino acids may comprise Asp in some embodiments. The PEG moiety may further comprise a functional group (e.g. an amine group, optionally an alkylamine group) at one or both ends of the PEG moiety that can react with the terminal residue of the peptide and/or the capture site (e.g. a biotin molecule) during synthesis to link the peptide and the capture site to one another. Thus, in particular embodiments, the indicator molecule comprises, consists essentially of or consists of the following structure:
(i) Cyc-_{D}Ala-Ser[Gal-Sial]-_{D}Ala-Arg-PEG-Biotin (SEQ ID NO: 11-PEG-Biotin)
(ii) Biotin-PEG-Asp-Glu-_{D}Ser-Nva-Cyc-_{D}Ala-Ser[Gal-Sial]-_{D}Ala-Arg-Phe-_{D}Ser-Val (Biotin-PEG-Asp-SEQ ID NO: 12)
(iii) Biotin-PEG-Asp-Arg-_{D}Ala-BIP-Ser-Pro-Ser[Gal-Sial]-_{D}Ala-_{D}Asp-Ser (Biotin-PEG-Asp-SEQ ID NO: 13)
(iv) Biotin-PEG-Asp-Ser-SEP-_{D}Ala-Ile-Orn-Ser[Gal-Sial]-_{D}Ala-Nle-Glu (Biotin-PEG-Asp-SEQ ID NO: 14)
(v) Biotin-PEG-Asp-_{D}Ala-Arg-Nva-_{D}Ser-ßAla-_{D}Ala-Nle-Ser[Gal-Sial]-BIP-Orn-_{D}Ala-Glu-Ser (Biotin-PEG-Asp-SEQ ID NO: 15); or
(vi) Biotin-PEG-Asp-Thr-_{D}Ala-Nle-Glu-_{D}Ala-Arg-ßAla-Cyc-Orn-_{D}Ser-Pro-Ser[Gal-Sial]-ßAla-Nva-_{D}Ser-Glu-Thr-Cha-_{D}Ser-Val (Biotin-PEG-Asp-SEQ ID NO: 16);
of which Cyc-_{D}Ala-Ser[Gal-Sial]-_{D}Ala-Arg-PEG-Biotin (SEQ ID NO: 11-PEG-Biotin) is particularly preferred.

Within the context of the present invention the indicator molecules (via the capture site) may bind to the capture molecules in a capture zone (as described in greater detail below) with relatively high affinity. In some embodiments, the dissociation constant (k_{d}) for the indicator molecule will be relatively low and preferably between 0M and 1 × 10⁻⁷M (depending on the sensitivity required of the assay). In certain embodiments of the invention, the dissociation constant for the indicator molecule will be between 1 × 10⁻¹⁵M and 1 × 10⁻⁹M.

In certain embodiments of the invention, such a binding interaction may be achieved as a result of direct binding of the capture site of the indicator molecule to the capture molecule present in the capture zone. In this context, direct binding means binding of the indicator molecule (via the capture site) to the capture molecule without any intermediary.

In preferred embodiments of the invention, the capture site of the indicator molecule and the capture molecule present in the capture zone are two halves of a binding pair. In this context, a binding pair consists of two molecules or entities capable of binding to each other. In certain embodiments of the invention, the binding interaction is specific such that each member of the binding pair is only able to bind its respective partner, or a limited number of binding partners. Moreover, as detailed above, it is preferable for the binding pair to exhibit relatively high affinity. The binding pair may be a binding pair found in nature or an artificially generated pair of interacting molecules or entities.

In some embodiments of the invention, the capture site of the indicator molecule and the capture molecule are two halves of a binding pair wherein the binding pair is selected from the following:- an antigen and an antibody or antigen binding fragment thereof; biotin and avidin, streptavidin, neutravidin or captavidin; an immunoglobulin (or appropriate domain thereof) and protein A or G; a carbohydrate and a lectin; complementary nucleotide sequences; a ligand and a receptor molecule; a hormone and hormone binding protein; an enzyme cofactor and an enzyme; an enzyme inhibitor and an enzyme; a cellulose binding domain and cellulose fibres; immobilised aminophenyl boronic acid and cis-diol bearing molecules; and xyloglucan and cellulose fibres and analogues, derivatives and fragments thereof.

In particular embodiments of the invention, the binding pair consists of biotin and streptavidin. In a further embodiment of the invention, the capture site of the indicator molecule comprises an epitope and the capture molecule comprises an antibody, which specifically binds to the epitope present at the first capture site. In the context of the present invention, the term antibody covers native immunoglobulins from any species, chimeric antibodies, humanised antibodies, F(ab')₂ fragments, Fab fragments, Fv fragments, sFv fragments and highly related molecules such as those based upon antibody domains which retain specific binding affinity (for example, single domain antibodies). The antibodies may be monoclonal or polyclonal. Thus, in specific embodiments, the capture molecule comprises an antibody. In other embodiments, the capture site comprises a biotin molecule and the capture zone comprises a streptavidin molecule.

Another core component of the enzyme detection devices, enzyme detection kits, and methods for detecting the presence in a test sample of cleavage activity of a sialidase enzyme described further herein is the binding molecule. The binding molecule is designed to specifically bind to the de-sialylated derivative of the indicator molecule formed following cleavage of the sialyl group from the indicator molecule by sialidase activity present in the sample. Formation of the de-sialylated derivative reveals a novel binding site to which the binding molecule can specifically bind. Thus, in preferred embodiments, the binding molecule cannot bind to the indicator molecule (at any appreciable or detectable level) unless and until cleavage of the sialyl group from the indicator molecule has occurred. In alternative embodiments, the binding molecule preferentially binds to the de-sialylated derivative over the sialylated peptide or indicator molecule. Thus, the binding molecule has a higher affinity for the de-sialylated derivative when compared with the sialylated form.

Alternatively viewed, in preferred embodiments, the binding molecule is capable of specifically binding to the de-sialylated indicator molecule (fragment) and is incapable of binding to the intact (sialylated) indicator molecule (at any appreciable or detectable level).

The skilled person is well able to determine whether or not a particular binding molecule binds to the de-sialylated derivative in preference over the sialylated form. This can, for instance, be expressed in terms of the relative dissociation constants. For example, in particular embodiments, the dissociation constant for the binding interaction between the binding molecule and the de-sialylated derivative may be 2-, 3-, 4-, 5-, 10-, 20-, 30-, 40-, 50-, 100-, 200-, 500-, 1000-fold (or more) lower than the dissociation constant for the binding interaction between the binding molecule and the sialylated form.

In all embodiments, the binding molecule comprises an antibody as defined in claim 7. For the avoidance of doubt, the term antibody covers native immunoglobulins from any species, chimeric antibodies, humanised antibodies, F(ab')₂ fragments, Fab fragments, Fv fragments, sFv fragments and highly related molecules such as those based upon antibody domains which retain specific binding affinity (for example, single domain antibodies). The antibody is monoclonal. The inventors have produced antibodies which only recognise the de-sialylated derivative of the indicator molecule and will therefore not bind to the indicator molecule (to any significant degree) unless and until cleavage of the sialyl group has occurred. Antibodies may be produced according to techniques known in the art. This may rely upon immunisation of an animal, such as a sheep, rabbit or goat, with the de-sialylated derivatives of the indicator molecules. Alternatively, the animal may be immunised with the de-sialylated peptides (i.e. without the capture site being attached). Monoclonal antibodies may be produced using well-known and characterised hybridoma technology.

Thus, in a related aspect, the invention also provides an antibody capable of specifically binding to the de-sialylated derivative of a
peptide comprising the following sequence:

X₁-X₂-X₃[Gal-Sial]-X₄-X₅ (SEQ ID NO: 17)

wherein:
Sial is a sialyl group;
X₃ is an amino acid comprising a glycosyl acceptor group and is selected from Ser, Thr, Tyr, Hyl, Hyp, Asn, Arg or phosphoserine (SEP), preferably wherein X₃ is Ser; and
X₁, X₂, X₄ and X₅ are independently selected from any amino acid provided that at least one, preferably at least two or three, of X₁, X₂, X₄ and X₅ is a _{D}-amino acid and/or a non-standard amino acid or a non-natural amino acid or an indicator molecule as provided herein, wherein the antibody binds preferentially to the de-sialylated derivative over the sialylated peptide or indicator molecule, and wherein the antibody has a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6. Thus, the antibody has a higher affinity for the de-sialylated derivative when compared with the sialylated form. The skilled person is well able to determine whether or not a particular antibody binds to the de-sialylated derivative in preference over the sialylated form. This can, for instance, be expressed in terms of the relative dissociation constants. For example, in particular embodiments, the dissociation constant for the binding interaction between the antibody and the de-sialylated derivative may be 2-, 3-, 4-, 5-, 10-, 20-, 30-, 40-, 50-, 100-, 200-, 500-, 1000-fold (or more) lower than the dissociation constant for the binding interaction between the antibody and the sialylated form. In particular embodiments, the antibody is incapable of binding to the sialylated peptide or indicator molecule. That is to say, it can only bind to the de-sialylated derivative after cleavage of the sialyl group has occurred.

Therefore, in a preferred embodiment, the antibody can specifically bind to the epitope: Cyc-_{D}Ala-Ser[Gal]-_{D}Ala-Arg-PEG-Biotin (de-sialylated form of SEQ ID NO: 11-PEG-Biotin); particularly to an epitope present in this molecule, more particularly an epitope present in the Gal peptide moiety of this molecule, Cyc-_{D}Ala-Ser[Gal]-_{D}Ala-Arg (de-sialylated form of SEQ ID NO: 11). Preferably, the antibody cannot bind (at any appreciable or detectable level) to the sialylated form of this molecule (Cyc-_{D}Ala-Ser[Gal-Sial]-_{D}Ala-Arg, i.e. SEQ ID NO: 11).

In another embodiment, an antibody is provided that can specifically bind to the epitope: Biotin-PEG-Asp-Glu-_{D}Ser-Nva-Cyc-_{D}Ala-Ser[Gal]-_{D}Ala-Arg-Phe-_{D}Ser-Val (de-sialylated form of Biotin-PEG-Asp-SEQ ID NO: 12); particularly to an epitope present in this molecule, more particularly an epitope present in the Gal peptide moiety of this molecule. Preferably, the antibody cannot bind (at any appreciable or detectable level) to the sialylated form of this molecule.

In a further embodiment, an antibody is provided that can specifically bind to the epitope: Biotin-PEG-Asp-Arg-DAla-BIP-Ser-Pro-Ser[Gal]-_{D}Ala-_{D}Asp-Ser (de-sialylated form of Biotin-PEG-Asp-SEQ ID NO:13); particularly to an epitope present in this molecule, more particularly an epitope present in the Gal peptide moiety of this molecule. Preferably, the antibody cannot bind (at any appreciable or detectable level) to the sialylated form of this molecule.

In a further embodiment, an antibody is provided that can specifically bind to the epitope: Biotin-PEG-Asp-Ser-SEP-_{D}Ala-Ile-Orn-Ser[Gal]-_{D}Ala-Nle-Glu (de-sialylated form of Biotin-PEG-Asp-SEQ ID NO: 14); particularly to an epitope present in this molecule, more particularly an epitope present in the Gal peptide moiety of this molecule. Preferably, the antibody cannot bind (at any appreciable or detectable level) to the sialylated form of this molecule.

In a further embodiment, an antibody is provided that can specifically bind to the epitope: Biotin-PEG-Asp-_{D}Ala-Arg-Nva-_{D}Ser-ßAla-_{D}Ala-Nle-Ser[Gal]-BIP-Orn-_{D}Ala-Glu-Ser (de-sialylated form of Biotin-PEG-Asp-SEQ ID NO: 15); particularly to an epitope present in this molecule, more particularly an epitope present in the Gal peptide moiety of this molecule. Preferably, the antibody cannot bind (at any appreciable or detectable level) to the sialylated form of this molecule.

In a further embodiment, an antibody is provided that can specifically bind to the epitope: Biotin-PEG-Asp-Thr-_{D}Ala-Nle-Glu-_{D}Ala-Arg-ßAla-Cyc-Orn-_{D}Ser-Pro-Ser[Gal]-ßAla-Nva-_{D}Ser-Glu-Thr-Cha-_{D}Ser-Val (de-sialylated form of Biotin-PEG-Asp-SEQ ID NO: 16); particularly to an epitope present in this molecule, more particularly an epitope present in the Gal peptide moiety of this molecule. Preferably, the antibody cannot bind (at any appreciable or detectable level) to the sialylated form of this molecule.

In all embodiments, the antibody has a heavy chain with 3 CDRs and a light chain with 3 CDRs, wherein the heavy chain CDR1 has SEQ ID NO: 1; the heavy chain CDR2 has SEQ ID NO: 2; the heavy chain CDR3 has SEQ ID NO: 3; the light chain CDR1 has SEQ ID NO: 4; the light chain CDR2 has SEQ ID NO: 5; and/or the light chain CDR3 has SEQ ID NO: 6.

The antibody may have a heavy chain that has SEQ ID NO: 7 and/or a light chain that has SEQ ID NO: 8.

Preferably, the antibody can specifically bind to an epitope present in the molecule Cyc-_{D}Ala-Ser[Gal]-_{D}Ala-Arg. Preferably, the antibody does not (at any appreciable or detectable level) bind to the molecule Cyc-_{D}Ala-Ser[Gal-Sial]-_{D}Ala-Arg.

For the (de-sialidated) molecule Cyc-_{D}Ala-Ser[Gal]-_{D}Ala-Arg-PEG-Oxime (de-sialylated form of SEQ ID NO: 11-PEG-Oxime) the antibody may have a K_{D} of less than 100 nM, preferably less than 80, 60, 50, 40, 30, 20 or 10 nM, for example about 7.9 nM. For this molecule it may have a Kₒₙ of about 54080M⁻¹s⁻¹; and a K_{off} of about 0.000427 s⁻¹.

The binding molecule may be directly or indirectly conjugated to (i.e. labelled with) a reporter molecule to permit detection of binding of the binding molecule to the indicator molecule. The reporter molecule may be any substance or moiety suitable for detection by any means available to those skilled in the art. Thus, the reporter molecule is typically capable of signal generation or production. In certain embodiments of the invention, the reporter molecule is selected from the following: - a gold particle; a chromogen; a luminescent compound; a fluorescent molecule; a radioactive compound; a visible compound; a liposome or other vesicle containing signal producing substances; an electroactive species; or a combination of enzyme and its substrate. A suitable enzyme-substrate combination for use as a reporter moiety may be the enzyme alkaline phosphatase and the substrate nitro blue tetrazolium-5-bromo-4-chloro-3-indolyl phosphate. In a particular embodiment of the invention, the reporter molecule is a gold particle.

Indirect labelling of the binding molecule with a reporter molecule is also envisaged within the present invention. Thus, the reporter molecule may be attached to a further binding molecule which in turn binds to the binding molecule to provide the label. This indirect binding may be mediated by an adaptor capable of simultaneously binding the binding molecule and the reporter molecule. As an illustrative embodiment, indirect labelling could be mediated by a further antibody that binds to the antibody binding molecule in specific fashion. The further antibody may be directly labelled with a reporter molecule such as a gold particle; a chromogen; a luminescent compound; a fluorescent molecule; a radioactive compound; a visible compound; a liposome or other vesicle containing signal producing substances; an electroactive species; or a combination of enzyme and its substrate. A suitable enzyme-substrate combination for use as a reporter moiety may be the enzyme alkaline phosphatase and the substrate nitro blue tetrazolium-5-bromo-4-chloro-3-indolyl phosphate. In a particular embodiment of the invention, the reporter moiety is a gold particle.

In embodiments where the reporter is a gold particle, the gold particle-binding molecule conjugate should be used an optical density of at least 4, preferably at least 5, 6 or 7, most preferably at least or about 8, 9 or 10.

In embodiments of the invention wherein the reporter molecule binds to the binding molecule by virtue of an adaptor molecule, the adaptor may be pre-complexed with the binding molecule prior to the addition of the test sample to the indicator molecule, provided that the adaptor does not prevent binding of the binding molecule to the cleaved indicator molecule.

The adaptor may be any material or molecule capable of mediating the indirect interaction of the binding molecule with the reporter molecule. In some embodiments, the adaptor is streptavidin and the binding molecule comprises a biotin molecule. The adaptor may also be an "adaptor binding pair" wherein said binding pair comprises:
(i) a first member capable of binding to the binding molecule; and
(ii) a second member capable of binding to the first member of the pair and to the reporter molecule. In certain embodiments of the invention, the detection region of the indicator molecule comprises biotin, the first member of the adaptor binding pair is avidin or streptavidin, the second member of the adaptor binding pair is biotin, and the reporter molecule comprises a moiety capable of binding biotin.

In view of the foregoing, in a complementary aspect, the de-sialylated derivatives of the peptides of the invention also form part of the invention and are used in the generation of the binding molecules, which are antibodies. Thus, the invention provides a peptide for use in generating an antibody as defined herein wherein the peptide is a de-sialylated derivative of a peptide according to the invention and the antibody is capable of specifically binding to the de-sialylated derivative of a peptide of the invention. Thus, in particular embodiments, the peptide is:
(i) Cyc-_{D}Ala-Ser[Gal]-_{D}Ala-Arg (de-sialylated form of SEQ ID NO: 11)
(ii) Asp-Glu-_{D}Ser-Nva-Cyc-_{D}Ala-Ser[Gal]-_{D}Ala-Arg-Phe-_{D}Ser-Val (de-sialylated form of SEQ ID NO: 12)
(iii) Asp-Arg-_{D}Ala-BIP-Ser-Pro-Ser[Gal]-_{D}Ala-_{D}Asp-Ser (de-sialylated form of SEQ ID NO: 13)
(iv) Asp-Ser-SEP-_{D}Ala-Ile-Orn-Ser[Gal]-_{D}Ala-Nle-Glu (de-sialylated form of SEQ ID NO: 14)
(v) Asp-_{D}Ala-Arg-Nva-_{D}Ser-ßAla-_{D}Ala-Nle-Ser[Gal]-BIP-Orn-_{D}Ala-Glu-Ser (de-sialylated form of SEQ ID NO: 15); or
(vi) Asp-Thr-_{D}Ala-Nle-Glu-_{D}Ala-Arg-ßAla-Cyc-Orn-_{D}Ser-Pro-Ser[Gal]-ßAla-Nva-_{D}Ser-Glu-Thr-Cha-_{D}Ser-Val (de-sialylated form of SEQ ID NO: 16).

A peptide comprising, consisting essentially of, or consisting of the sequence Cyc-_{D}Ala-Ser[Gal]-_{D}Ala-Arg is particularly preferred.

In particular embodiments, the peptide may be conjugated to a carrier protein in order to improve immunogenicity and thus antibody production in the host organism. For instance, the peptide may be conjugated to keyhole limpet hemocyanin. The carrier protein may be at the N- or C-terminus of the peptide.

In view of the foregoing, the invention further provides enzyme detection devices, enzyme detection kits, and methods for detecting the presence in a test sample of cleavage activity of a sialidase enzyme incorporating an indicator molecule, capture molecules and binding molecules as defined above. The use of binding molecules, such as antibodies, that bind only to the de-sialylated derivative of the indicator molecule but not to the uncleaved indicator molecule, enable detection of sialidase activity at low concentrations in test samples.

Thus, in a further aspect, the invention provides an enzyme detection device, or enzyme detection kit for detecting the presence in a test sample of cleavage activity of a sialidase enzyme, the device comprising:
(i) an indicator molecule
   comprising
   (a) a peptide comprising the following sequence:

      X₁-X₂-X₃[Gal-Sial]-X₄-X₅ (SEQ ID NO: 17)

      wherein:
      Sial is a sialyl group;
      X₃ is an amino acid comprising a glycosyl acceptor group and is selected from Ser, Thr, Tyr, Hyl, Hyp, Asn, Arg or phosphoserine (SEP), preferably wherein X₃ is Ser; and
      X₁, X₂, X₄ and X₅ are independently selected from any amino acid provided that at least one, preferably at least two or three, of X₁, X₂, X₄ and X₅ is a _{D}-amino acid and/or a non-standard amino acid or a non-natural amino acid; and
   (b) a capture site which remains intact following cleavage of the sialyl group from the indicator molecule by a sialidase enzyme present in the sample;
(ii) a capture zone to receive the test sample, wherein the capture zone comprises capture molecules capable of binding to the capture site of the indicator molecule, irrespective of whether or not the indicator molecule has been cleaved, in order to immobilise the indicator molecule; and
(iii) binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred; and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6.

The invention further provides a method for detecting the presence or absence in a test sample of cleavage activity of a sialidase enzyme, the method comprising:
(i) bringing an indicator molecule as provided herein into contact with the test sample;
(ii) adding to the test sample binding molecules as provided herein capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred;
(iii) capturing the de-sialylated derivative of the indicator molecule at a capture zone through binding of capture molecules in the capture zone to the capture site, said capture molecules being able to bind to the capture site irrespective of whether or not the indicator molecule has been cleaved; and
(iv) detecting cleavage of the sialyl group from the indicator molecule by determining binding of the binding molecules to the de-sialylated derivative of the indicator molecule captured in the capture zone.

The devices, kits, and methods of the invention have been shown by the inventors to have specific application in the field of diagnosis of BV. Thus, the invention further provides a method for diagnosing bacterial vaginosis in a test sample by detecting cleavage activity of a sialidase enzyme in the sample, the method comprising:
(i) bringing an indicator molecule as provided herein into contact with the test sample;
(ii) adding to the test sample binding molecules as provided herein capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred;
(iii) capturing the de-sialylated derivative of the indicator molecule at a capture zone through binding of capture molecules as defined herein in the capture zone to the capture site, said capture molecules being able to bind to the capture site irrespective of whether or not the indicator molecule has been cleaved; and
(iv) detecting cleavage of the sialyl group from the indicator molecule by determining binding of the binding molecules to the de-sialylated derivative of the indicator molecule captured in the capture zone wherein an increased level of cleavage compared to a control diagnoses bacterial vaginosis.

In particular embodiments, the indicator molecule may be (pre-)immobilised in the capture zone via the capture molecules (i.e. prior to contact with the test sample). Thus, the invention also provides a method for detecting the presence or absence in a test sample of cleavage activity of a sialidase enzyme, the method comprising:
(i) adding the test sample to a capture zone comprising capture molecules, said capture molecules as defined herein being bound to the capture site of an indicator molecule as provided herein wherein said capture molecules remain bound to the capture site irrespective of whether or not cleavage of the sialyl group from the indicator molecule by sialidase enzyme present in the sample occurs;
(ii) adding binding molecules as provided herein capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred;
(iii) detecting cleavage of the sialyl group from the indicator molecule by determining binding of the binding molecules to the de-sialylated derivative of the indicator molecule captured in the capture zone.

Similarly, the invention also provides a method for diagnosing bacterial vaginosis in a test sample by detecting cleavage activity of a sialidase enzyme in the sample, the method comprising:
(i) adding the test sample to a capture zone comprising capture molecules as defined herein, said capture molecules being bound to the capture site of an indicator molecule as provided herein wherein said capture molecules remain bound to the capture site irrespective of whether or not cleavage of the sialyl group from the indicator molecule by sialidase enzyme present in the sample occurs;
(ii) adding binding molecules as provided herein capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred;
(iii) detecting cleavage of the sialyl group from the indicator molecule by determining binding of the binding molecules to the de-sialylated derivative of the indicator molecule captured in the capture zone wherein an increased level of cleavage compared to a control diagnoses bacterial vaginosis.

Consequently, for those embodiments in which the indicator molecule is (pre-)immobilised in the capture zone via the capture molecules (i.e. prior to contact with the test sample), prior to step (i) of the method, the indicator molecule may be added to the capture zone such that the indicator molecule is bound in the capture zone via the capture molecules. Addition of the binding molecules may occur simultaneously with or after the test sample has been added to the capture zone. Thus, steps (i) and (ii) of the method may occur sequentially or simultaneously.

In the context of the methods provided herein, the step of "adding" binding molecules to the test sample should be understood to encompass any step that brings the binding molecules into contact with the test sample. Thus, this step may encompass a step of applying the test sample to a device comprising the binding molecules. The binding molecules may be in solution, or may be on a carrier. For example, the binding molecules may be dried onto or otherwise impregnated into or onto a solid support, which may be the "conjugate pad" discussed elsewhere herein. In preferred embodiments the test sample is brought into contact with a solid support onto which the binding molecules have been dried. Liquid comprised in the test sample and/or added to the carrier allows the binding molecules to resolvate.

In embodiments where the binding molecules are dried onto or otherwise impregnated into or onto a solid support, the solid support preferably comprises or consists of fibreglass, polyester fibres, or a material having similar properties. The solid support should preferably have one or more of the following properties: basis weight around 75g/m²; caliper about 0.38 - 0.43 mm; wicking rate about 3-5 (s/2 cm); and/or water absorption about 63-79 mg/cm². Thus, the conjugate pad may have these properties. Similarly, the sample pad may have these properties.

Whilst in preferred embodiments the binding molecules are incapable of binding to the indicator molecule unless and until cleavage of the sialyl group from the indicator molecule by sialidase enzyme present in the sample has occurred, in some embodiments, the binding molecules preferentially bind to the de-sialylated derivative of the indicator molecule over the sialylated form as described elsewhere herein. Thus, some degree of binding to the sialylated indicator molecule may occur (this may be considered background signal in some cases). However, because the binding molecules will preferentially bind to the de-sialylated derivative a much greater signal is generated in samples comprising the de-sialylated derivative relative to samples in which the sialylated indicator molecule has not been cleaved or no indicator molecule is present.

In order to take into account background levels of sialidase activity (if present), the methods typically involve comparing measured levels of cleavage in the test sample to a control. Typically, the control represents corresponding levels of sialidase activity in a healthy subject. By "healthy subject" is meant a subject not suffering from BV. The control may be in a corresponding test sample taken from a matched healthy control. Alternatively, the control may be a threshold level of sialidase activity set by determining sialidase activity in a range of healthy and diseased patients. Suitable methods for setting a threshold are well known to those skilled in the art. The threshold may be mathematically derived from a training set of patient data. The score threshold thus separates the test samples according to presence or absence of BV. The interpretation of this quantity, i.e. the cut-off threshold may be derived in a development or training phase from a set of patients with known outcome. The threshold may therefore be fixed prior to performance of the claimed methods from training data by methods known to those skilled in the art.

For example, a cube reader may be used, for example the Optricon reader from OpTricon GmbH (Chembio Diagnostic systems) of Schwarzschildstrasse 1, D-12489 Berlin, Germany), as demonstrated in the Examples. By way of example, in the assays used in the examples a cube reading of less than 10 correlates with the absence of a visual signal and is considered to be a negative result and therefore indicative of the absence of bacterial vaginosis; and a cube reading of at least 30 units correlates with a strong visual signal and is considered to be a positive result and therefore indicative of the presence of bacterial vaginosis.

A cube reading of 10-20 units correlates with a faint visual signal and is considered to be a result that may be indicative of the absence of established bacterial vaginosis, but may be indicative of a low level of infection, for example the early-stage of bacterial vaginosis.

In particular embodiments, the sialidase enzyme to be detected originates from Prevotella, Bacteroides and/or Mobiluncus species and/or *Gardnerella vaginalis.*

The enzyme detection devices of the invention may be supplied in a format ready for immediate use. Alternatively, the essential components may be provided as a kit of parts, optionally together with suitable reagents and/or instructions for assembly of the enzyme detection device. Accordingly, in another aspect, the invention provides an enzyme detection kit for detecting the presence in a test sample of cleavage activity of a sialidase enzyme, the kit comprising:
(i) an indicator molecule as provided herein for adding to the test sample;
(ii) capture molecules as defined herein capable of binding to the capture site of the indicator molecule, irrespective of whether or not the indicator molecule has been cleaved;
(iii) a solid support to which the capture molecules can be attached to form a capture zone to receive the test sample; and
(iv) binding molecules as provided herein capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred.

In related aspects, the invention also provides for use of an enzyme detection device as provided herein for diagnosing BV in a test sample. Similarly, the invention also provides for use of a method as provided herein for diagnosing BV in a test sample. The invention further provides for use of an enzyme detection kit as provided herein for diagnosing BV in a test sample.

The invention may be performed in lateral flow or vertical flow devices in certain embodiments. Generally, therefore, the invention relies upon some form of solid support. The solid support may define a liquid flow path for the sample. In specific embodiments, the solid support comprises a chromatographic medium or a capillary flow device. The invention may be provided in a test strip format in some embodiments.

In specific embodiments of the invention, the capture zone is formed on a solid support. Any support to which the capture molecules may be attached to form a capture zone is intended to be encompassed. The solid support may take the form of a bead (e.g. a sepharose or agarose bead) or a well (e.g. in a microplate) for example. Thus, in certain embodiments the device comprises a solid support to which the capture molecules are attached to form the capture zone. In the case of the kits of the invention, the solid support may be provided without the capture molecules attached. In those embodiments, the user of the kit may immobilize the capture molecules on the solid support to form the capture zone prior to use of the device with a test sample. The kit may, therefore, also comprise means for immobilizing the capture molecules on the solid support. The immobilizing means may comprise any suitable reagents to permit the capture zone to be formed. The solid support may be pre-formed with suitable immobilizing means. For example, the solid support may comprise biotin molecules arranged to interact with avidin (e.g. streptavidin) molecules that form (part of) the capture molecules. Of course, other binding pair interactions may be used to immobilize the capture molecules on the solid support to form a capture zone, as discussed herein and as would be readily understood by one skilled in the art.

The capture zone may be defined by the immobilization therein or thereon of capture molecules capable of binding to the capture site of indicator molecules. Immobilization of capture molecules may be achieved by any suitable means. Wherein the device is a flow device comprising a chromatographic medium, the capture molecules may be immobilized by directly binding to the medium or immobilized indirectly via binding to a carrier molecule, such as a protein, associated with, or bound to, the medium.

In further embodiments, the solid support further comprises a sample application zone to which the sample is applied. The sample application zone may be pre-loaded with the indicator molecule, such that when the test sample is applied any enzyme in the sample acts upon the cleavage site of the indicator molecule within the sample application zone. The sample application zone may contain a barrier, which holds the sample in the sample application zone for a pre-determined period of time. This permits the sample to interact with the indicator molecule for a sufficient period to achieve measurable levels of cleavage. This may be 1, 2, 3, 4, 5, 6, 7, 8, 9, 10, 15, 20, 30, 60 minutes or more depending upon the sialidase enzyme to be detected, as would be readily understood by one skilled in the art. About 5-15 minutes, 5-10 minutes or about 5 minutes is preferred. The barrier may be degraded by the sample, or otherwise removed, after this period of time thus allowing the sample to continue to flow through the device. Alternatively, the test sample and indicator molecule may be pre-mixed or pre-incubated prior to adding the mixture to the device, such as to the sample application zone. However, where the test sample and indicator molecule may be pre-mixed or pre-incubated it is possible to omit the sample application zone. Here, it may be possible to add the mixture directly to the capture zone to permit immobilization of the indicator molecules through interaction with the capture molecules. In some embodiments, the test sample may be applied to the chromatographic medium at a site upstream from the capture zone such that it is drawn, for example by capillary action, through the capture zone. The chromatographic medium may be made from any material through which a fluid is capable of passing, such as a fluidic channel or porous membrane. In certain embodiments of the invention, the chromatographic medium comprises a strip or membrane, for example a nitrocellulose strip or membrane.

The indicator molecule may be present on a carrier, which should be an inert material and preferably porous. It may be a suitable polymer, such as Polytetrafluoroethylene. For example, the carrier may be an inert porous membrane disc. The indicator molecule may, for example, have been freeze-dried onto the carrier. In preferred embodiments, the test sample and indicator molecule may be pre-mixed or pre-incubated prior to adding the mixture to the device. This may involve contacting the sample with a carrier comprising the indicator molecule, preferably in freeze-dried form. This may, for example, be done in a container such as a tube. Preferably, the sample and indicator molecule are incubated for at least 3, 4 or 5 minutes and no more than 30 minutes, preferably for about 5-15 minutes, 5-10 minutes or about 5 minutes, for example 3-7 or 4-6 minutes.

The skilled person will be able to determine suitable amounts and concentrations of the various components, but by way of example for a single assay or in a single kit the indicator molecule is preferably present in an amount of at least 30, 40, 50, 60, 70, 80, 90 or 100 ng, more preferably at least or about 110, 120, 130, 140, 150, 160, 170, 180 or 190 ng.

The binding molecules must be provided in the device in a manner that permits interaction with the indicator molecule, if the sialyl group is cleaved from the indicator molecule by sialidase enzyme present in the sample. The binding molecules may, therefore, be pre-mixed with the indicator molecules prior to application to the device. This may be before or after the indicator molecules have been mixed with the test sample. It is preferably after to avoid any effect the binding molecules may have on enzyme activity (in the test sample) at the cleavage site of the indicator molecule. The binding molecules can preferably be provided on or in the device at any point upstream of the capture zone, such that the binding molecules encounter the test sample and indicator molecules before the indicator molecules are immobilised (via interaction between the capture site of the indicator molecule and capture molecules defining the capture zone). Alternatively, the binding molecule may be added to the capture zone after the test sample and indicator molecules have been added to the capture zone. This ensures that any indicator molecule will already be immobilized at the capture zone, providing (in the case of cleaved (i.e. de-sialylated) indicator molecule) a binding site for the binding molecules to produce a signal.

The solid support may further comprise a control zone, downstream of the capture zone in relation to sample flow, and the sample application zone if present, containing further binding molecules, referred to herein as "control detection binders" which bind to the binding molecules to indicate successful completion of an assay using the device. Alternatively, the further binding molecules (control detection binders) may bind to a further molecule, referred to herein as "control detection molecules" added to the sample or to the device and which flows with the sample through the device. The further molecule (control detection molecule) may be labelled, either directly or indirectly, with a reporter molecule as defined herein. Preferably, the reporter molecule is the same reporter molecule as attached to the binding molecules, for ease of detection, although it may be different. The control zone is spatially separated from the capture zone, for example to produce two separate test lines if the reporter is bound or immobilized in each respective zone. This control zone is used to confirm that the test sample, including the binding molecules, has passed through the entire device and confirms that the device is operating correctly. A positive signal is expected at the control zone independent of whether sialidase activity is present in the sample or not. The further binding molecules are selected based upon the nature of the binding molecules which bind to the cleavage site of the indicator molecules or on the nature of the further molecule added to the sample. The binding molecules and further binding molecules or further molecules and further binding molecules may form a binding pair as defined herein. For example, if the binding molecule is a species specific antibody (e.g. a sheep antibody), the further binding molecule may be an anti-species antibody (e.g. an anti-sheep antibody). For example, the control detection molecule may be a chicken IgY antibody and the control detection binder may be an anti-chicken IgY antibody, or *vice versa.* Alternatively, if the further molecule is an antibody from a different species, e.g. a chicken or a goat, the further binding molecule may be an appropriate anti-species antibody. This permits immobilization of the binding molecule or further molecule at the control zone by virtue of a specific interaction. The further binding molecules may be immobilized in the control zone by any suitable means, for example by a covalent or noncovalent interaction.

According to all aspects of the invention, the test sample may be a vaginal sample, optionally a vaginal swab. The test sample may be collected by any suitable means and presented in any form suitable for use with the present invention. Typically, the sample is collected using a sterile swab. Moreover, as part of obtaining the test sample from its original source, the sample may undergo one or more processing or pre-treatment steps prior to testing using the invention. In one embodiment, a sample may be processed so as to produce a solution or suspension for testing. Moreover, in certain embodiments, the test sample may be stored, for example frozen at around -20 °C, as a means of preserving the sample for any given length of time prior to testing using the invention.

It should be noted that the invention is typically performed *in vitro* based upon isolated samples. The methods of the invention may include steps of obtaining a sample for testing in some embodiments, e.g. using a sterile swab.

### BRIEF DESCRIPTION OF THE DRAWINGS

The invention will now be described by way of example with respect to the accompanying drawings in which:
Figure 1 is a schematic view of one format of the assay in accordance with the invention. The format relies upon the following basic components: a solid support (1); a capture molecule (2); an indicator molecule comprising a capture site (3) and a peptide of the invention, the peptide comprising a Gal-Sial cleavage site (4); and a binding molecule (5) that binds to the indicator molecule only after cleavage (6) has occurred. The indicator molecule Cyc-_{D}Ala-Ser[Gal-Sial]-_{D}Ala-Arg-PEG-Biotin (SEQ ID NO: 11-PEG-Biotin; also termed "MOL600c" herein) is shown by way of example.
Figure 2 is a schematic view of an enzyme detection device in accordance with the present invention and shows operation of the device in the absence (Fig. 2A) or presence (Fig. 2B) of sialidase activity.
Figure 3 shows the visual read-out of the assay (shown in Figure 2) as levels of sialidase activity in the test sample are increased.
Figure 4 is a schematic view of an enzyme detection device in accordance with the present invention. The figure specifies the exact longitudinal dimensions and position of each of the card components.
Figure 5 is a schematic view of the two conjugation chemistries used to couple peptides of the invention to Keyhole Limpet Hemocyanin (KLH): (A) cysteine/maleimide coupling; (B) hydrazine/benzaldehyde coupling.
Figure 6 shows some of the non-standard and non-natural amino acids employed during the design of the peptides of the invention.
Figure 7 is a schematic view summarising the synthetic approach used to produce peptides of the invention.
Figure 8 shows the production of a sialylated peptide of the invention via an enzymatic route using transialidase and fetuin (a sialic acid donor).
Figure 9 shows various peptides and peptide-protein conjugates synthesised as part of the invention. (A) Tabular summary of the peptides and peptide-protein conjugates synthesised. (B) Schematic overview of the peptides and peptide-protein conjugates synthesised.
Figure 10 shows the initial ELISA results following immunisation of sheep with KLH-peptide conjugates of the invention. (A) a schematic of the detection method; (B)-(D) the results from the first bleeds, indicating that all sheep responded to the immunisations. Numbers 1056, 1057, 1058, 1059, 1062, 1063, 1064, 1065, 1066 and 1067 designate antisera from a specific immunised sheep respectively.
Figure 11A-C shows ELISA results from a second bleed of the sheep described in Figure 10. Antisera showed an increase in response in the second bleeds compared to the first bleeds
Figure 12A-B shows all three bleeds compared for sheep CF1062-1067.
Figure 13 shows ELISA results when antisera CF1064 was evaluated against MOL136 and MOL136c. (A) a schematic of the detection method; (B) ELISA results showing significant specificity to the galactosyl (i.e. de-sialylated) peptide MOL136 in preference to the sialylated peptide MOL136c.
Figure 14 shows detection of the de-sialylated peptide MOL136 using gold-labelled CF1064 via lateral flow assay with little/no signal observed in relation to the sialylated peptide MOL136c nor the negative control (in which no peptide was included in the sample).
Figure 15 shows detection of the de-sialylated peptide MOL136 using gold-labelled CF1064 and CF1065 via lateral flow assay with little/no signal observed in relation to the sialylated peptide MOL136c nor the negative control (in which no peptide was included in the sample).
Figure 16 shows a lateral flow assay comparing MOL136 and MOL136c binding of gold-labelled CF1064 in the presence of healthy vaginal swab extracts.
Figure 17 shows a lateral flow assay of MOL136c in the presence of 25 U/ml sialidase.
Figure 18 shows lateral flow data showing a comparison between different affinity purified antisera fractions. (A) Graphical representation of line intensities; (B) lateral flow test strips as observed: (1) - Gold-sensitised affinity purified polyclonal antibody, (2) - Gold-sensitised affinity purified polyclonal antibody with cross-reactive antibodies removed, (3) Gold-sensitised cross-reactive antibodies from affinity purification.
Figure 19 shows (A) a comparison of MOL136, MOL136c, MOL600 and MOL600c binding by ELISA and (B) lateral flow experiments showing marked improvement of performance as compared with MOL136/136c (refer to Figure 18).
Figure 20 shows stability data for heat dried MOL600c in polypropylene tubes at different storage temperatures and with two different heat drying methods: a speed vac and a heat block method. The measurement in ELISA are normalised to percentage desialylation as this would result in recognition by the antibody and increase in signal.
Figure 21 shows typical data for MOL600c batches. (A) an analytical HPLC trace; (B) postive ion electrospray MS confirmation.
Figure 22 is a schematic showing the column methods used for antibody purification with different sepharose matrices. The orange tags shown in respect of MOL600 and MPL600c represent biotin. The purple tag shown in respect of MOL615 represents an oxime group.
Figure 23 shows stability data of lateral flow cassettes in storage over 12 weeks at 37 ºC.
Figure 24 shows a lateral flow standard curve of sialidase concentration incubated for 5 minutes with peptide MOL600c. The test lines were quantified by using the Cube Reader.
Figure 25 shows lateral flow testing of a healthy volunteer vaginal sample on a flock swab. The sample was extracted in 1ml of sample buffer and split five ways. (A) sample but no peptide; (B) sample with MOL600c; (C) sample with MOL600c and sialidase at 500U/ml for 5 minutes; (D) sample with MOL600c spun down; (E) sample with MOL600c and the same sialidase incubation spun down.
Fig. 26 shows the chemical formula of MOL616.
Fig. 27 shows results of Example 13 showing the specificity of antibody 125.1 for MOL600 compared to the sialylated form, MOL600c.
Fig. 28 shows a comparison of assays A and B using a range of different sialidase concentrations. For each sialidase concentration, the left bar represents the cube reading for assay B and the right bar represents the cube reading for assay A.
Fig. 29 shows a comparison of assays A and B using a range of different sialidase concentrations and read times. For each sialidase concentration, the bar represent from left to right (i) the cube reading for assay A after 5 minute read time; (ii) the cube reading for assay A after 10 minute read time; (iii) the cube reading for assay B after 5 minute read time; and (iv) the cube reading for assay B after 10 minute read time.

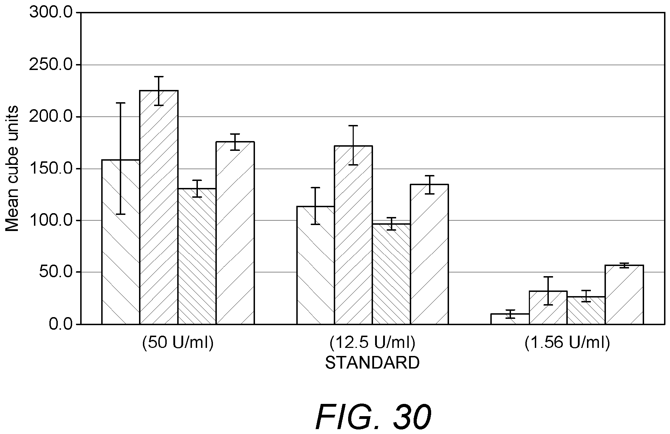

Fig. 30 shows a comparison of assays A, A', B and B' using a range of different sialidase concentrations, indicator concentrations and read times.. For each sialidase concentration, the bar represent from left to right (i) the cube reading for assay B with 1µg/ml indicator molecule per disc and 5 minutes read time; (ii) the cube reading for assay B with 3µg/ml indicator molecule per disc and 5 minutes read time; (iii) the cube reading for assay A with 1µg/ml indicator molecule per disc and 10 minutes read time; and (iv) the cube reading for assay A with 3µg/ml indicator molecule per disc and 10 minutes read time.
Fig. 31 shows the sequences of antibody 125.1 indicating the CDR and framework regions.

### DESCRIPTION OF PREFERRED EMBODIMENTS

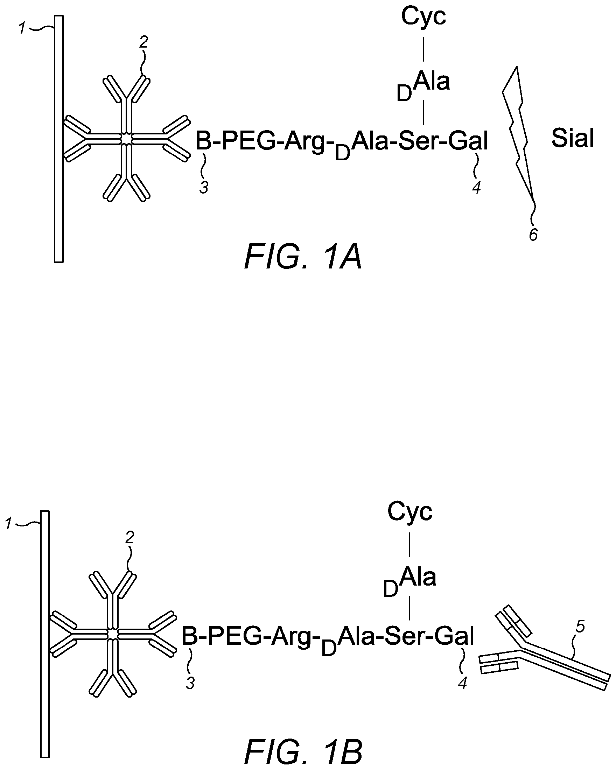

Figure 1 is a schematic view of one format of the assay in accordance with the invention. The format relies upon the following basic components: a solid support (1); a capture molecule (2); an indicator molecule comprising a capture site (3) and a peptide of the invention, the peptide comprising a Gal-Sial cleavage site (4); and a binding molecule (5) that binds to the indicator molecule only after cleavage (6) has occurred. The indicator molecule Cyc-_{D}Ala-Ser[Gal-Sial]-_{D}Ala-Arg-PEG-Biotin (also termed "MOL600c" herein) is shown by way of example.

In the format shown, the capture molecule (2) is streptavidin. Here, the capture molecule (2) binds to a biotin capture site (3) within the indicator molecule.

As shown in Fig. 1A, once the indicator molecule of the invention is added to a test sample, sialidase enzyme present in the sample specifically recognises the Gal-Sial cleavage site (4) and cleaves the sialyl group from the indicator molecule (6).

As shown in Fig. 1B, this cleavage event (6) produces a binding site for the specific antibody binding molecule (5). The binding molecule (5) is unable to bind to the indicator molecule until cleavage (6) has occurred. Thus, the antibody binding molecule (5) binds to the amino acid sequence Cyc-_{D}Ala-Ser[Gal]-_{D}Ala-Arg produced as a result of cleavage of the sialyl group. The antibody binding molecule (5) does not bind to the Cyc-_{D}Ala-Ser[Gal-Sial]-_{D}Ala-Arg sequence prior to cleavage (not shown).

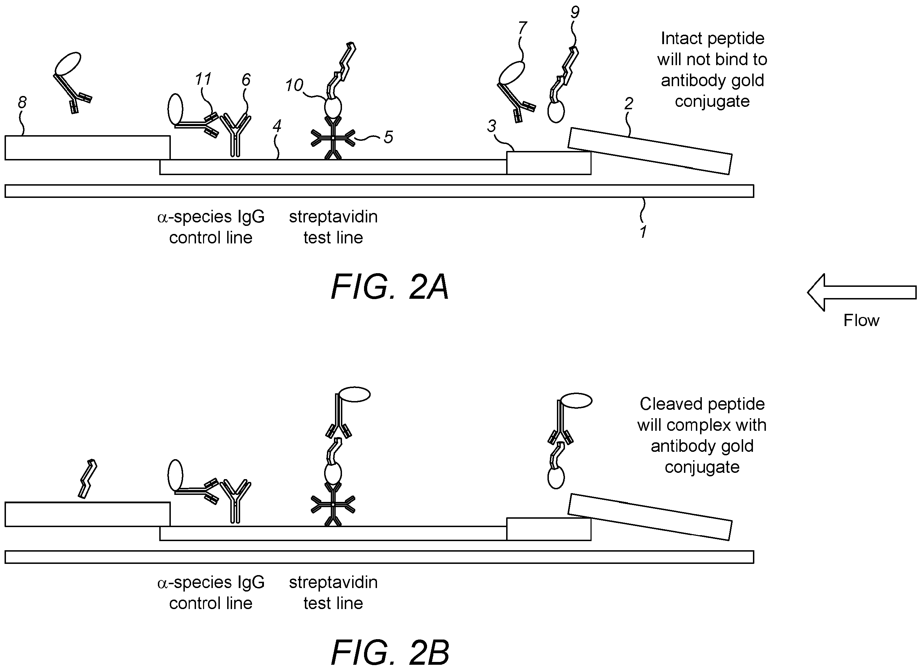

Figure 2 is a schematic view of an enzyme detection device in accordance with the present invention and shows operation of the device in the absence (Fig. 2A) or presence (Fig. 2B) of sialidase activity. The test strip includes an adhesive liner (1) upon which the other components of the device are assembled. From right to left, the sample application zone (2) is in the form of an absorbent pad. This is laid partially overlapping the conjugate pad (3), which is impregnated with the labelled binding molecules (7). In alternative embodiments, the labelled binding molecules may be impregnated in the sample application zone and this removes the need for a separate conjugate pad. The conjugate pad (3) is in fluid connection with a nitrocellulose membrane (4). The nitrocellulose membrane (4) contains immobilized streptavidin molecules (5) which define a capture zone. The membrane (4) further contains immobilized further binding molecules (6) downstream of the capture zone which bind to further labelled molecules (11) which pass through the device with the sample and form a separate control zone. Alternatively, the immobilised further binding molecules may bind to labelled binding molecules (7). The device optionally further comprises an absorbent pad (8) to absorb any test sample and reagents reaching the end of the device.

In use, the indicator molecule (9) is added to the test sample prior to bringing the test sample into contact with the sample application zone (8) of the device. As shown in Figure 2A, in the absence of sialidase activity in the test sample, the sialyl group is not cleaved from the indicator molecule (9). Upon sample flow into the conjugate pad (3), the binding molecules (7) are unable to bind to the indicator molecule (9) because cleavage of the sialyl group has not occurred. The indicator molecules become bound at the capture zone via the interaction between streptavidin (5) and the biotin capture site (10) of the indicator molecule (9). The labelled binding molecules (7) are not immobilized at the capture zone because they cannot bind to the indicator molecules (9). Accordingly, the labelled binding molecules flow through to the control zone and beyond. Further labelled molecules (11) also pass through the device to the control zone where they are immobilized by binding to the immobilized further binding molecules (6). Thus, absence of sialidase activity is displayed as a signal only at the control zone, but not at the capture zone. Excess sample, potentially containing labelled binding molecules (7), flows into the absorbent pad (8).

As shown in Fig. 2B, in the presence of sialidase activity in the test sample, the sialyl group is cleaved from the indicator molecule (9). Upon sample flow into the conjugate pad (3), the binding molecules (7) are able to bind to the indicator molecule (9) because cleavage of the sialyl group has occurred. The indicator molecules become bound at the capture zone via the interaction between streptavidin (5) and the biotin capture site (10) of the indicator molecule (9). The labelled binding molecules (7) are immobilized at the capture zone due to binding to the de-sialylated indicator molecules (9) at the cleavage site. Due to the relative excess of labelled binding molecule (7) to binding sites at the capture zone some labelled binding molecules (7) still flow through to the control zone and beyond. Further labelled molecules (11) also pass through the device to the control zone where they are immobilized by binding to the immobilized further binding molecules (6). Thus, presence of sialidase activity is displayed as a signal both at the capture zone and the control zone. Excess sample flows into the absorbent pad (8).

It should be noted that the control zone is optional. The presence or absence of sialidase activity in the sample can be monitored solely based upon the presence or absence of a corresponding signal at the capture zone.

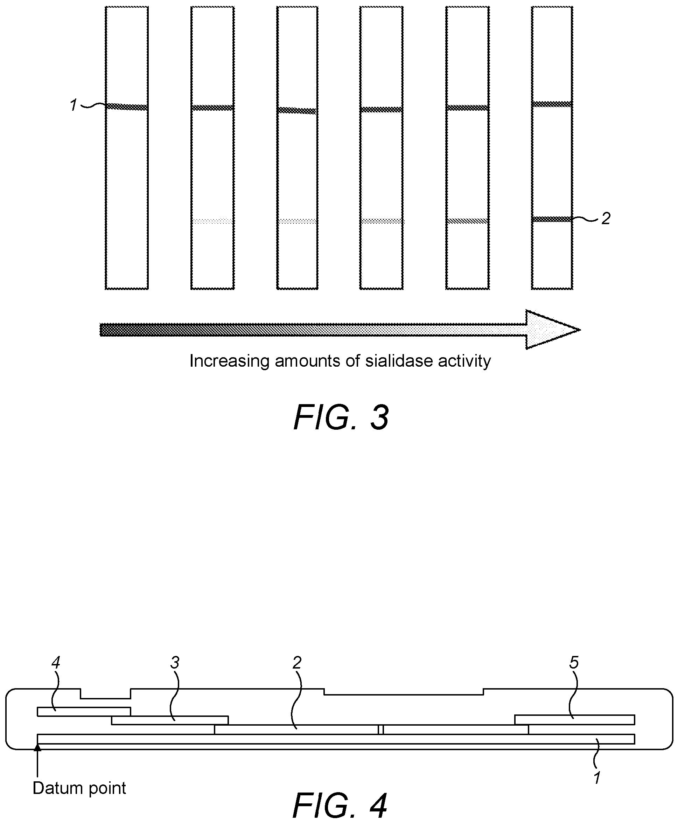

Figure 3 shows the visual read-out of the assay (shown in Figure 2) as levels of sialidase activity in the test sample are increased. As can readily be seen, the signal at the control zone (1) is constant as sialidase amounts increase. In contrast, as sialidase amounts increase, the signal at the capture zone (2) also increases. This is due to cleavage of the sialyl group from the indicator molecule at the cleavage site by sialidase activity. This reveals a binding site, enabling binding of the binding molecules which is detected at the capture zone (2) via interaction between capture molecules defining the capture zone and the capture site of the indicator molecules.

Figure 4 is a schematic view of one specific enzyme detection device in accordance with the present invention. The table below provides a legend for the figure and specifies the exact longitudinal dimensions and position of each of the card components in this particular embodiment. Of course, the dimensions and positions may be varied as would be readily understood by one skilled in the art.

| **Component** | **Size** | **Position from Datum point** |
|---|---|---|
| Backing card (1) | 60mm | 0mm |
| Nitrocellulose Membrane (2) | 25mm | 20mm |
| Conjugate Pad (3) | 17mm | 5mm |
| Sample Pad (4) | 10mm | 0mm |
| Absorbent Pad (5) | 22mm | 38mm |

The invention will be further understood with reference to the following experimental examples.

### EXAMPLES

### Example 1: Assay Chemistry Principle and experimental design

In summary, the principle works on the basis of antibody recognition of the chemical product of the sialidase reaction, where the chemical substrate is a peptide designed specifically to react with sialidase and the product of the reaction is then recognised by the antibody raised to that synthetic product. As shown in Figures 1 and 2, a glycopeptide containing sialic acid and with a biotin tag is provided. When contacted with a test sample containing sialidase activity, the sialyl group is cleaved from the glycopeptide by sialidase to expose the pendant galactosyl group on the peptide. An antibody raised against the de-sialylated product then specifically binds the cleaved product. By using an antibody-gold conjugate and a lateral flow strip with a streptavidin test line the presence of the de-sialylated product can be detected as a red line and a direct measure of the sialidase activity in a sample.

Five peptides were originally designed and the subsequent antibodies raised were evaluated and profiled as to their performance in a lateral flow assay. It was shown that under optimised conditions that some of the antibodies outperformed others in terms of their cross reactivity to the substrate peptide and their overall signal levels in the assay. In evaluations described below for the feasibility phase of the project the peptides were reevaluated and the peptide/antibody combination with best performance was taken forward into further development.

### Example 2: Peptide Design

In the design of the candidate peptide sequences a number of factors were taken into account:

### Size

As a general rule, molecules with molecular weight (MW) less than 5000 daltons are unlikely to stimulate a good immune response in a host organism. The peptides were planned as relatively short sequences with the intention of conjugating to KLH (Keyhole Limpet Hemocyanin) which results in a much more effective immunogen. A range of different lengths (9-20 amino acids) were proposed to cover any potential variation in performance.

### Conjugation Chemistry

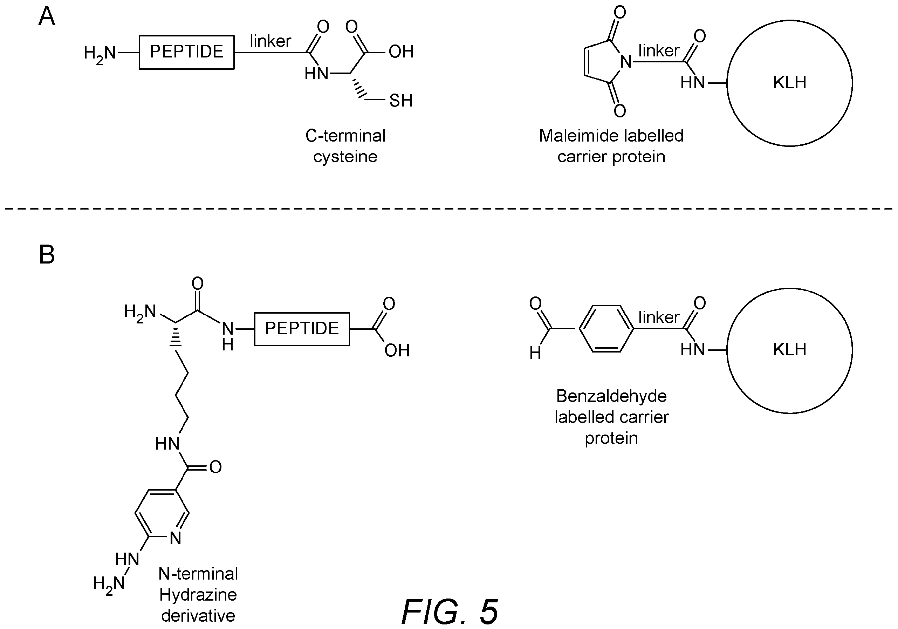

Two different conjugation chemistries were used. For two of the peptides a cysteine label was incorporated into the structure so that it could be conjugated to carrier proteins using a standard maleimide based chemistry. For the other three peptides a relatively new hydrazine based chemistry was used which is a more controllable process than cysteine chemistry as there is less risk of oxidation of the peptide prior to coupling and the extent of coupling can be easily monitored by UV absorbance. An overview of these conjugation chemistries is shown in Figure 5.

### Position of the Galactose Sugar

The aim was to obtain antibodies with very high affinity to the galactose sugar and surrounding regions of the peptide. With this in mind it was decided to position the sugar centrally in the structure so that it was well flanked by other distinctive peptide features. This approach would hopefully minimise peripheral binding antibodies which lacked interaction with the sugar moiety.

### Structural Diversity

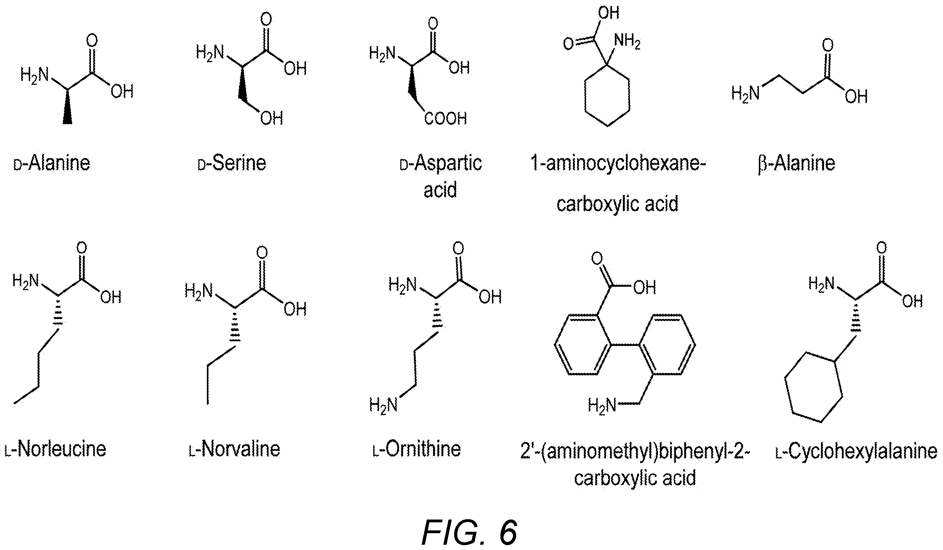

A range of amino acids were used in constructing the various peptide sequences. By combining charged, hydrophilic and hydrophobic groups along the sequence diverse topologies would be promoted. "Hinge groups" such as β-Alanine were also employed to allow additional degrees of freedom in the overall structural fold. In addition to this unnatural amino acids were employed to add to the sequence diversity and to promote an immune response. To reduce susceptibility to proteases some D-amino acids were also incorporated. Some of the non-standard and non-natural amino acids employed are shown in Figure 6.

After consideration of these factors the following peptide sequences were chosen as putative epitopes.

| **Peptide sequence** | **Designated name** |
|---|---|
| ---Arg-_{D}Ala-Bip-Ser-Pro-Ser(β-Gal)-_{D}Ala-_{D}Asp-Ser---- | MOL133 (Desialylated SEQ ID NO: 13) |
| ---Ser-SEP-_{D}Ala-Ile-Orn-Ser(β-Gal)-_{D}Ala-Nle-Glu---- | MOL134 (Desialylated SEQ ID NO: 14) |
| --- DAla-Arg-Nva-DSer-βAla-DAla-Nle-Ser(β-Gal)-Bip-Orn-DAla- Glu-Ser---- | MOL135 (Desialylated SEQ ID NO: 15) |
| ---Glu-_{D}Ser-Nva-Cyc-_{D}Ala-Ser(β-Gal)-_{D}Ala-Arg-Phe-_{D}Ser-Val--- | MOL136 (Desialylated SEQ ID NO: 12) |
| ---Thr-DAla-Nle-Glu-DAla-Arg-βAla-Cyc-Orn-DSer-Pro-Ser(β- Gal)-βAla-Nva-DSer-Glu-Thr-Cha-DSer-Val---- | MOL137 (Desialylated SEQ ID NO: 16) |

Spacers, conjugation groups and biotin labels were added to the C or N termini.

### Example 3: Peptide Synthesis

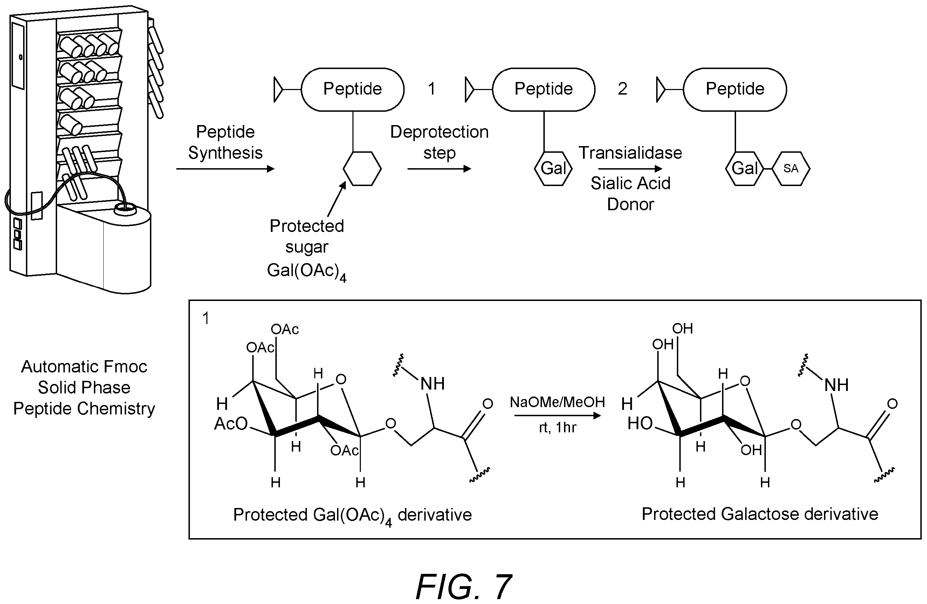

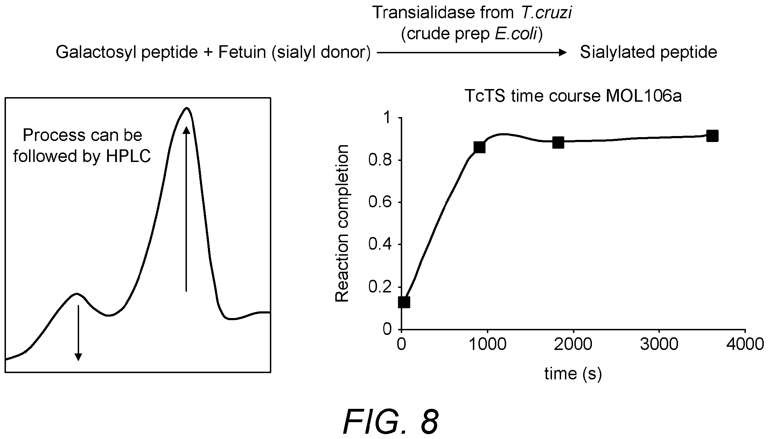

Galactose-labelled peptides were synthesised using solid phase chemistry methods on an automated microwave synthesiser. All peptides were purified using reverse phase HPLC and characterised by Electrospray LCMS. To label the galactose moiety with sialic acid an enzymatic route was employed using recombinant transialidase from *T. cruzi* (TcTS) and fetuin as a sialic acid donor. Figures 7 and 8 summarise the synthetic approach used.

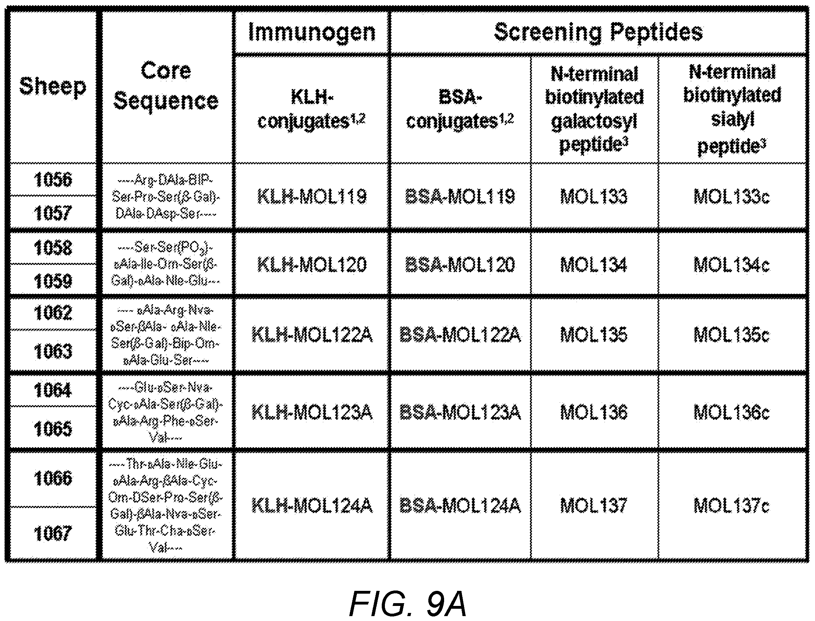

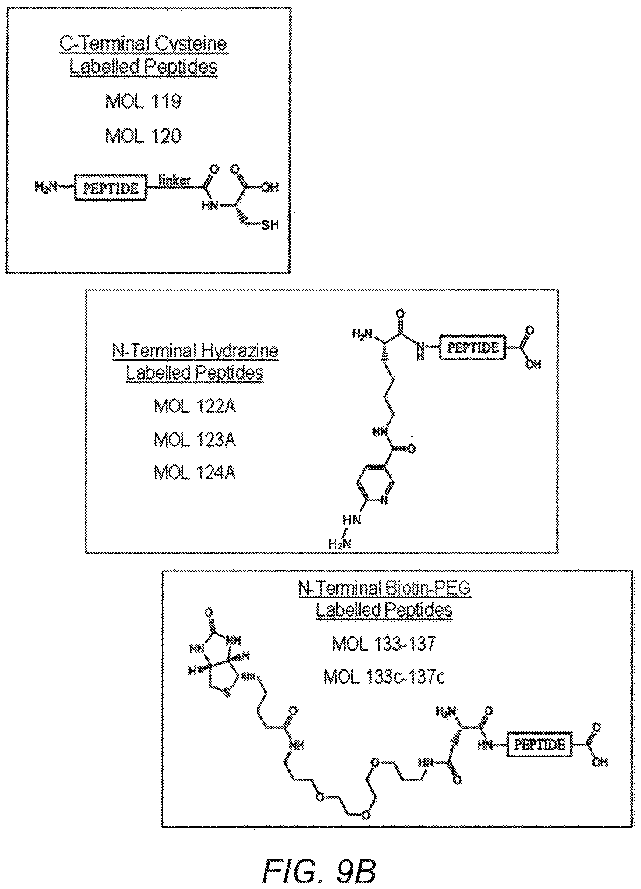

As well as the relevant peptide immunogens for conjugation to carrier proteins, two biotinylated derivatives of each sequence were also synthesised, one labelled with sialic acid. These biotinylated derivatives would form the basis for the lateral flow assay format. Figure 9 shows the peptides synthesised for this work according to the various immunisations. Peptides labelled with sialic acid are designated with a 'c'; e.g. MOL136c. Peptides which lack the sialyl group lack this designation (e.g. MOL136).

### Example 4A: Antibody generation

### Immunisations

All five peptide immunogens were coupled to KLH and BSA using the appropriate coupling chemistries (see Figure 9). The KLH conjugates were submitted to Micropharm Ltd for immunisation into ten sheep (two per peptide). The program was initiated with a dose of 1 mg of KLH-peptide in PBS. Three further boosters of 0.5 mg were given over a period of three months. Three separate bleeds were taken during this period (one sample and two production bleeds) and supplied to Mologic. BSA conjugates were retained to carry out the screening of the various bleeds.

### Initial ELISA Screens

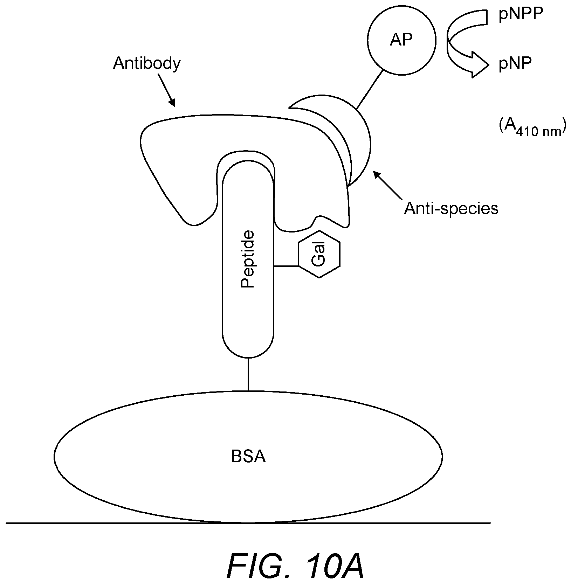

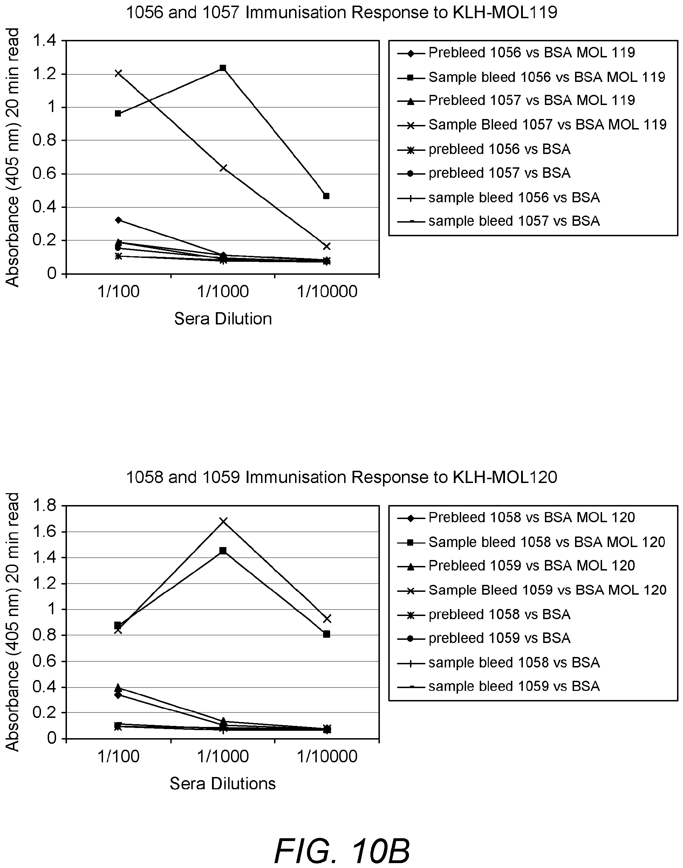

Polystyrene high binding multi-well plates were sensitized with the appropriate BSA conjugate and non-conjugated BSA as a control. Sera samples were incubated in the BSA blocked wells at various dilutions and further bound with a secondary anti-sheep antibody conjugated to alkaline phosphatase (AP). Incubations with para-Nitrophenylphosphate (pNPP) AP substrate indicated the presence of any binding. In all cases buffer, BSA and pre-bleed controls resulted in no background binding to the plate. Figure 10A shows a schematic of the detection method. Figures 10B-10D show the results from the first bleeds, indicating that all sheep responded to the immunisations. Sera dilutions at 1/1000 gave high signal whilst controls of BSA were negative, confirming peptide specific response. Pre-bleed controls were also negative.

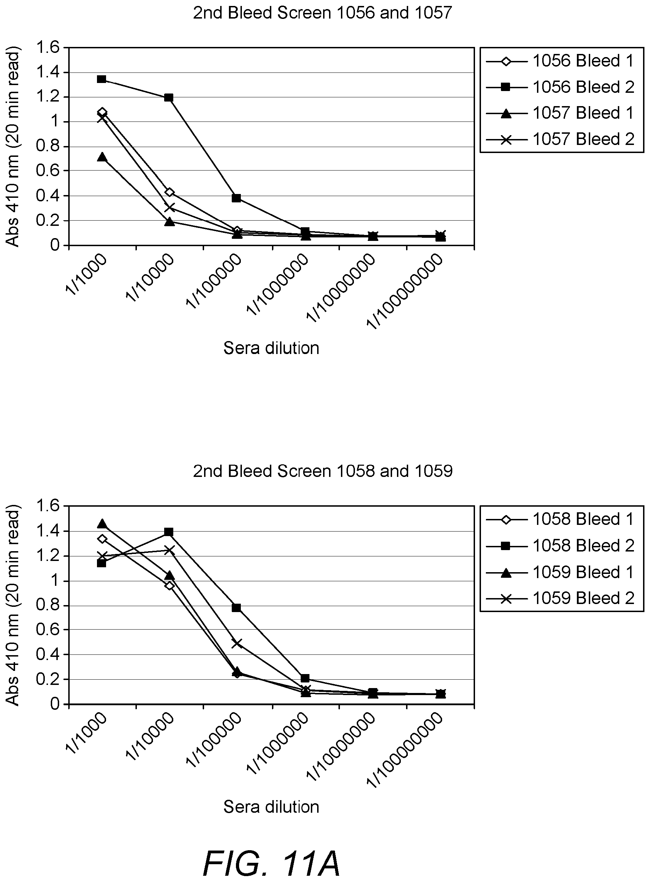

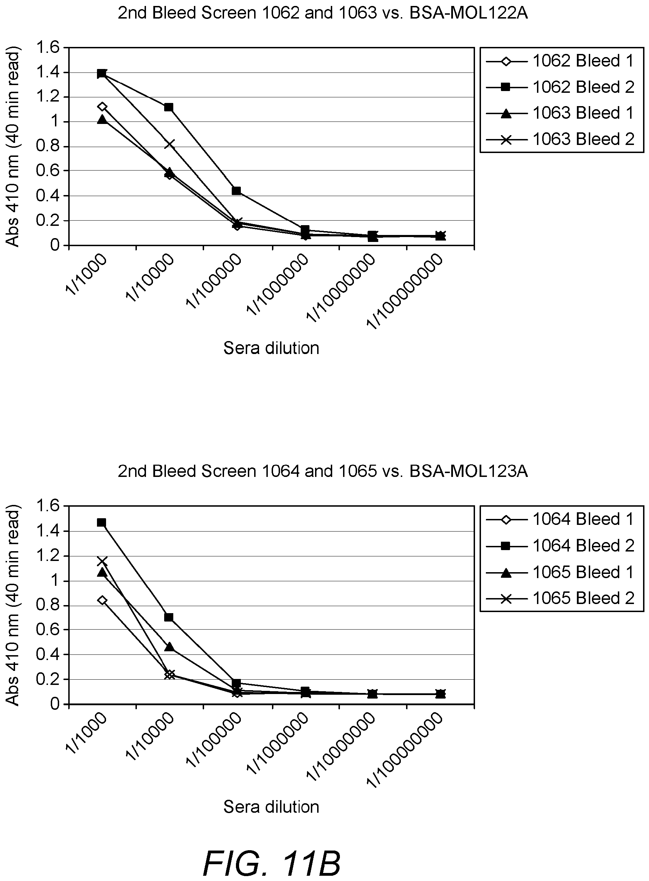

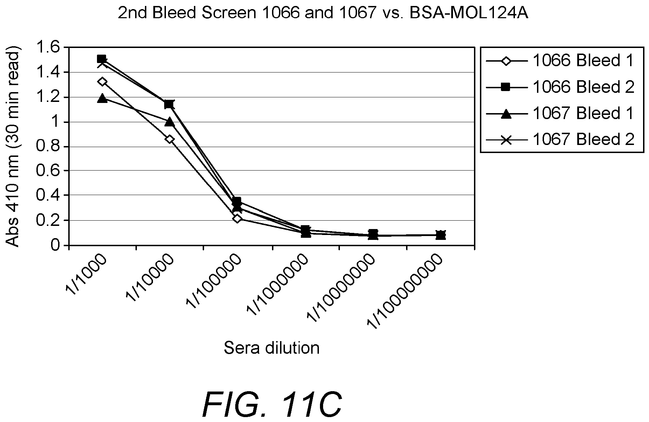

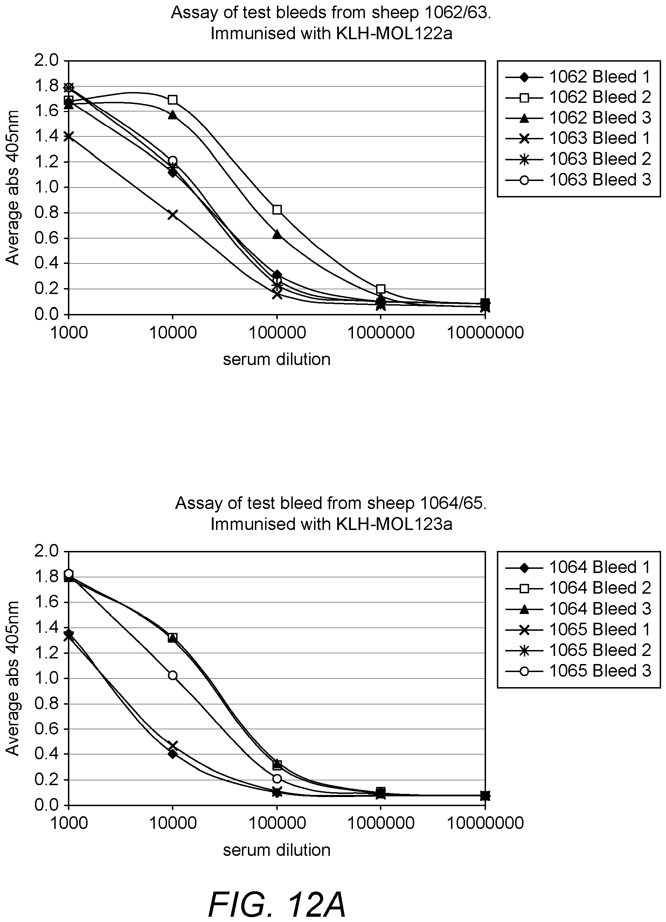

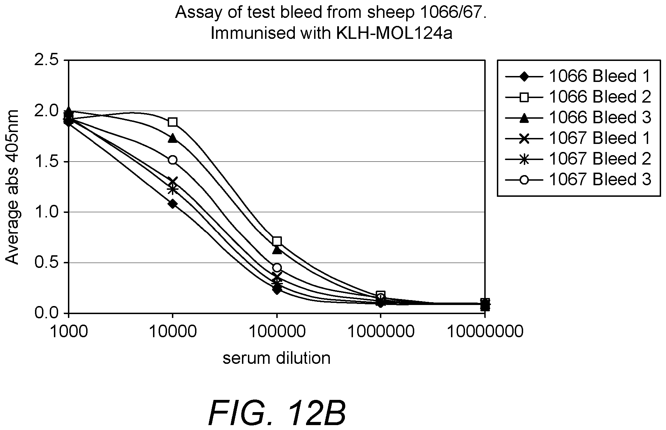

As further bleeds became available, these were also analysed by ELISA showing an enhancement of response over time. Figure 11 illustrates the relationship between the first two bleeds for all the sheep while Figure 12 shows all three bleeds compared for sheep CF1062-1067. Interestingly the third bleeds appear plateaued or even reduced in terms of their overall response to antigen. Nevertheless in some cases a binding response was detected at 1/1000000 dilution suggesting a polyclonal response highly sensitive to the peptide. Despite the slightly lower response in the third bleeds, these were preferred for affinity purifications as they were the most likely to contain a sub-population of highly specific antibodies.

### Example 4B: Antisera CF1064 and CF1065 and detection of MOL136 and MOL136c

Immunogen : KLH-MOL123A
Biotinylated Peptides: MOL136 (galactosyl); MOL136c (sialyl)

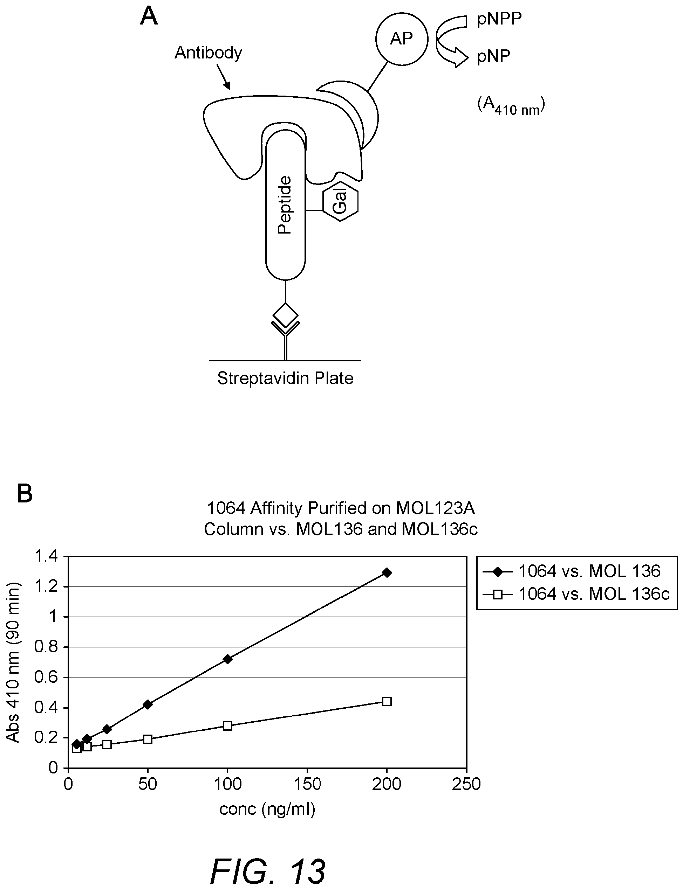

Both sheep responded well to immunisation with KLH-MOL123A with CF1064 giving a slightly stronger response overall (see Figure 11B). In ELISA format (a schematic of which is shown in Figure 13A), binding of CF1064 was evaluated against MOL136 and MOL136c, showing significant specificity to the galactosyl (i.e. de-sialylated) peptide MOL136 in preference to the sialylated peptide MOL136c (Figure 13B). Thus, excellent discrimination of the two peptides by antisera CF1064 is demonstrated.

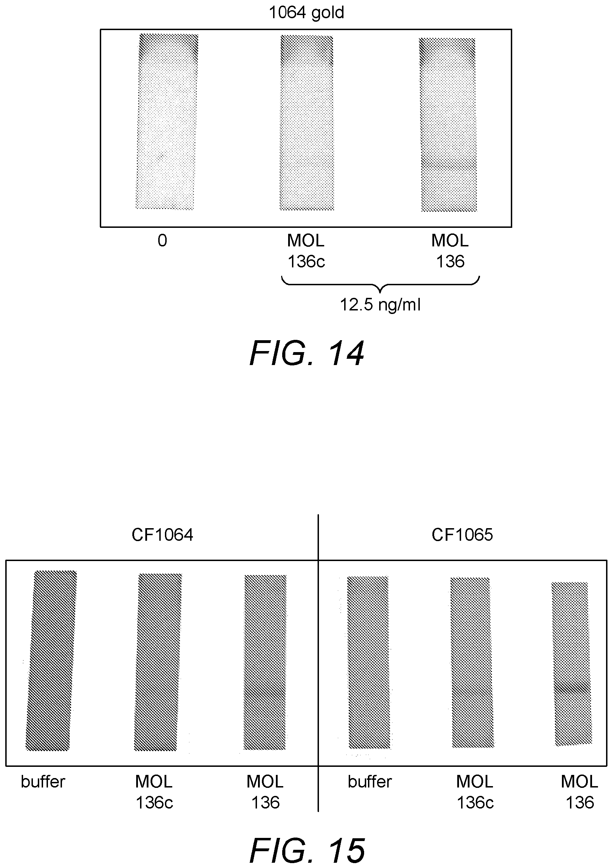

Similar properties were observed in lateral flow format. CF1064 was conjugated to gold particles in optimised conditions (15 µg/ml loading in 10 mM sodium borate buffer; pH 8.0) and assayed against MOL136 and MOL136c at a final concentration of 1.3 ng/ml (38 pg per strip). Thus, a capture zone was first formed on each lateral flow test strip using 1.5 mg/ml streptavidin. Then, 3 µl of each peptide (12.5 ng/ml peptide in PBST) was mixed with 10 µl of gold-labelled CF1064 and 15 µl of running buffer (0.5M Tris at pH 7.5 + 3% BSA + 0.5% Triton X-100) before each sample was run along a separate lateral flow test strip. A negative control in which no peptide was added was also run as a negative control. 15 µl of running buffer wash was then run along each lateral flow test strip. De-sialylated peptide MOL136 was clearly detected in the capture zone, observed as a visible line on the test strip. Conversely, little/no signal was observed in relation to the sialylated peptide MOL136c nor the negative control (see Figure 14).

Similar results were observed using gold-labelled CF1065, with a higher signal level observed for CF1065 relative to CF1064 (see Figure 15).

### Example 5: Detection of MOL136 and MOL136c using CF1064 in the presence of healthy vaginal swab extracts

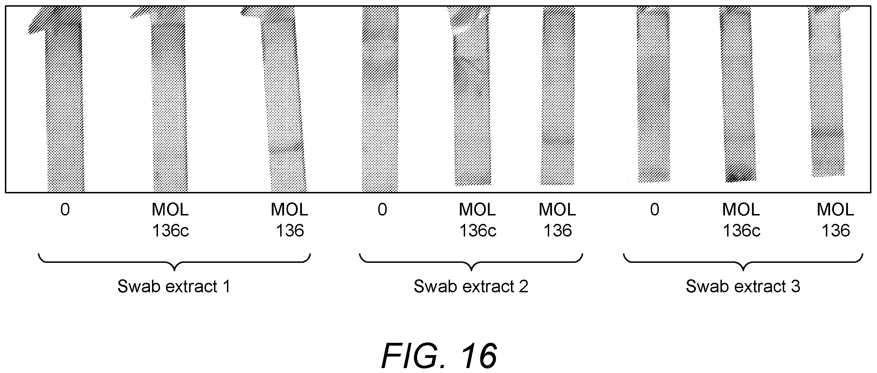

The procedure described in Example 4B was repeated in the presence of healthy vaginal swab extracts. Although the signal levels were slightly lower, the specificity to MOL136 was retained without any interference of the swab matrix (see Figure 16).

### Example 6: Detection of sialidase activity using MOL136c and CF1064

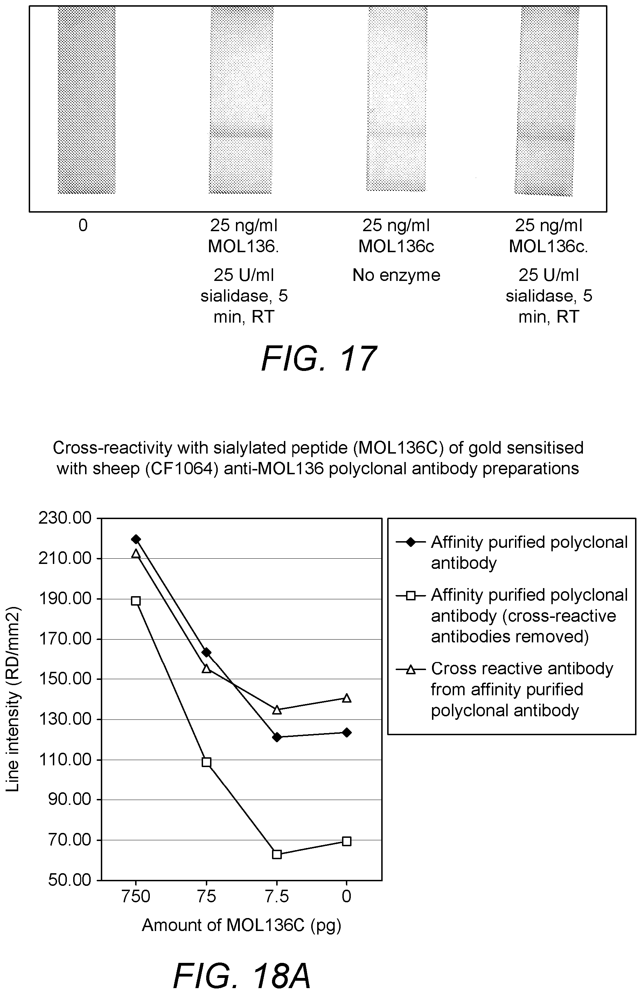

The procedure described in Example 4B was repeated in the presence of 25 U/ml sialidase. A sample in which sialidase was absent was used as a negative control. A further negative control was run in which no peptide and no sialidase was included in the sample. As a positive control, MOL136 was used in place of MOL136c in the presence of 25 U/ml sialidase. A clear positive signal was observed in respect of MOL136c incubated in the presence of 25 U/ml sialidase in PBST for 5 minutes. The level of signal was similar in intensity to that of the positive control which shows the putative maximum signal. As expected, little/no signal was observed in respect of the negative controls (see Figure 17).

Overall both CF1064 and CF1065 showed good specificity for the galactosyl (i.e. de-sialylated) peptide MOL136 and are both potential candidates for monoclonal screening.

### Example 7: Peptide development

After an evaluation of the peptides and antisera, it was shown that sera CF1064 and peptides MOL136 and MOL136c provided the most feasible combination for the test. Sheep CF1064 was immunised with the same peptide sequence as MOL136 but as a KLH conjugate using cysteine-maleimide chemistry in place of the biotin.

MOL136: biotin-PEG-Asp-Glu-_{D}Ser-Nva -Cyc-_{D}Ala-Ser[Gal]-_{D}Ala-Arg-Phe-_{D}Ser-Val-OH MOL136c: biotin-PEG-Asp-Glu-_{D}Ser-Nva-Cyc-_{D}Ala-Ser[Gal-Sial]-_{D}Ala-Arg-Phe-_{D}Ser-Val-OH

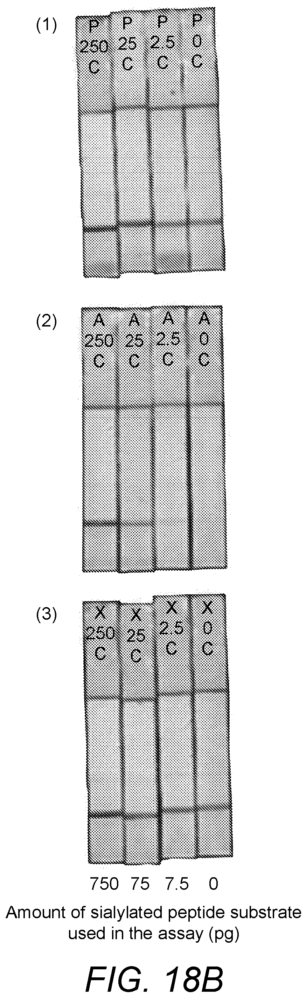

To purify the antisera, a method was devised using a streptavidin column to capture MOL136 and MOL136c. Antibody was then bound and eluted from the MOL136 column and absorbed to the MOL136c column to remove any peripheral binders picking up common epitopes between the two structures. By evaluating the purified antibody in lateral flow, the overall specificity to the original galactosyl peptide antigen and the degree of cross-reactivity to the sialylated peptide was measured (Figure 18). This is important because it represents the amount of non-specific binding observed in a negative result in the prototype and, without an optical reader in use, would need to be reduced significantly so that the naked eye would not pick it up.

Purified and absorbed fraction performed best in the assay when compared to non-absorbed materials. Despite the significantly lower background signal, further method development was required to improve the performance by truncating the peptide structure.

To refine the peptide design and remove peripheral interactions of antibodies not binding the galactose moiety in the peptide, the original sequence MOL136 was truncated to a shorter sequence MOL600:
MOL600: NH₂-Cyc -_{D}Ala-Ser[Gal]-_{D}Ala-Arg-PEG-Biotin
MOL600c: NH₂-Cyc-_{D}Ala-Ser[Gal-Sial]-_{D}Ala-Arg-PEG-Biotin

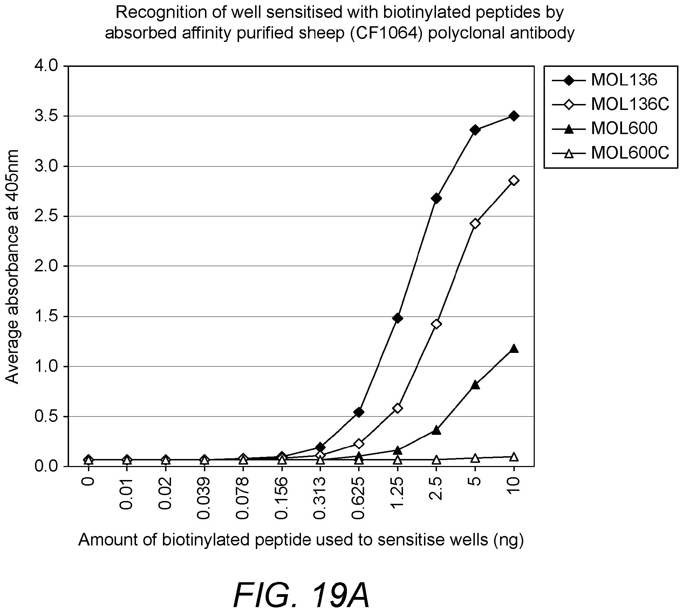

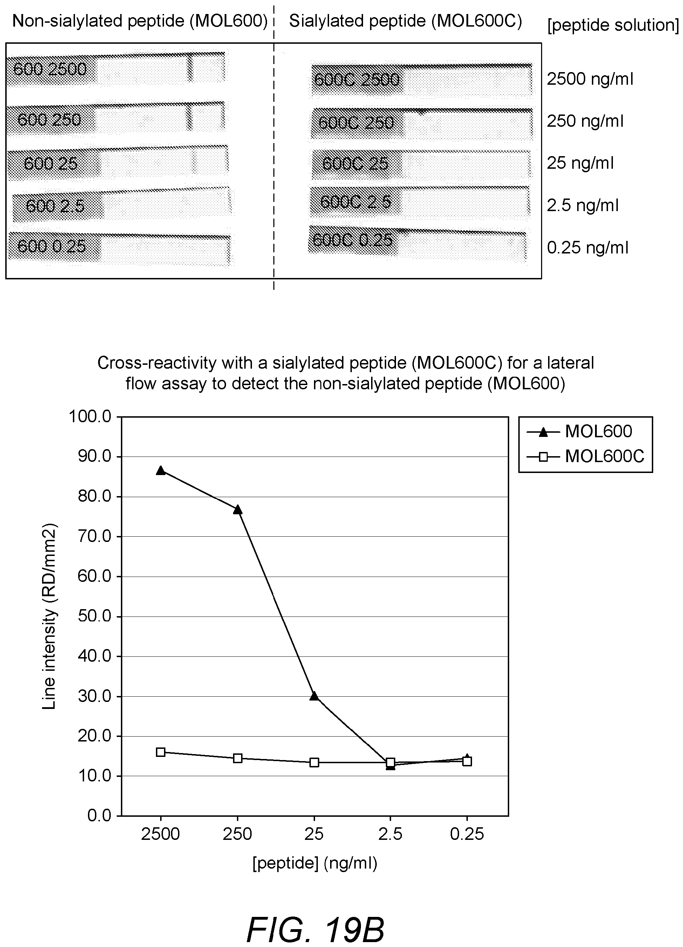

MOL600 and MOL600c were then assayed by ELISA and lateral showing a significant improvement in performance (see Figure 19).

### Example 8: MOL600c formulation/stability studies and scale-up production

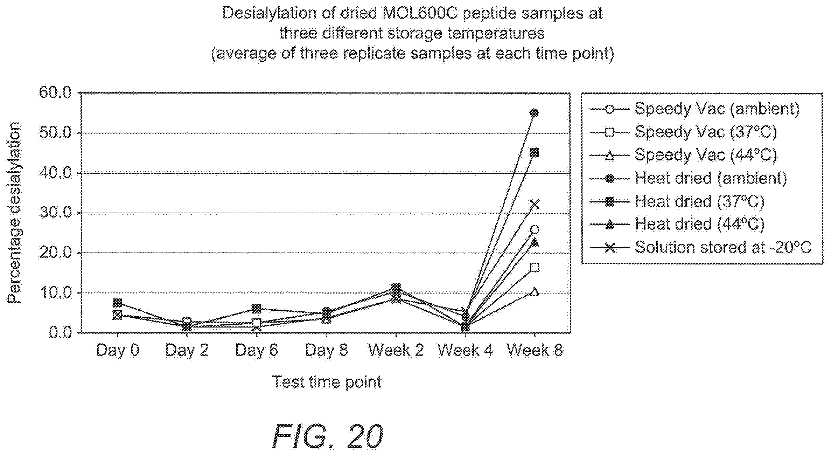

For evaluation of how the MOL600c peptide could be formulated, a study was set up to look at drying conditions. The peptide was formulated in a drying buffer, air dried and stored at room temperature prior to use in the prototype format. Data is shown in Figure 20 demonstrating the stability of peptide MOL600c stored in polypropylene tubes at different temperatures and with different drying methods. The peptides retained their functionality up to week 4 overall, however performance appears to decline at 8 weeks.

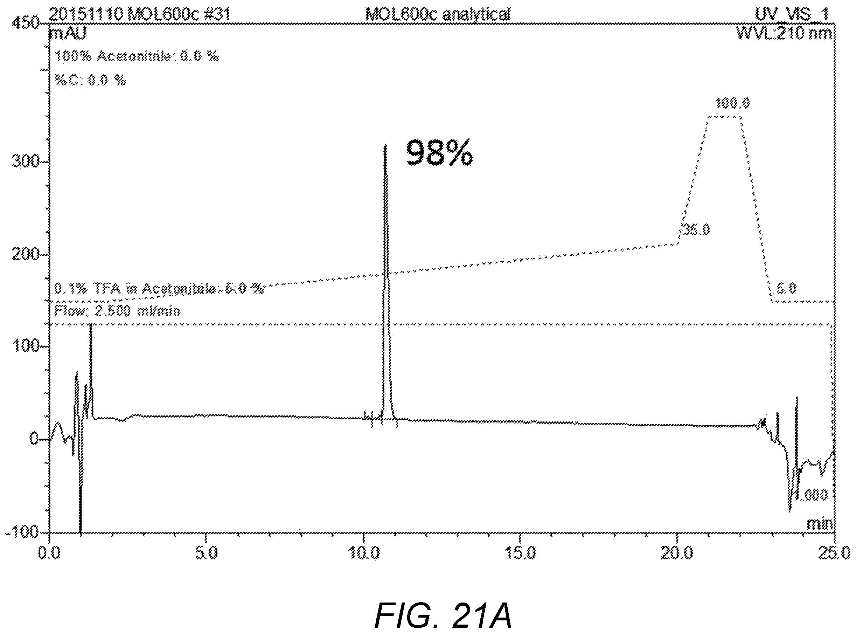

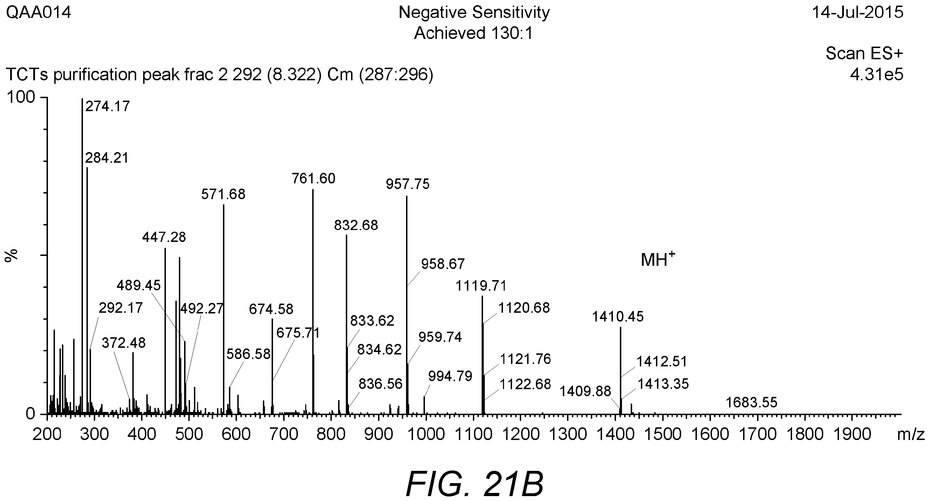

For manufacture, the peptide synthesis has been shown to be scalable and can be ramped up to manufacturing batches. The peptide is produced by solid phase synthesis on an automated system and then purified by HPLC. Two further chemical steps are required and a further final purification by HPLC to a specification of >95% purity. The product is confirmed by electrospray mass spectrometry. As only 12 ng of peptide is required per test, 1-2 mg batches of peptide are of adequate size. Figure 21 shows typical analytical data of a completed batch of MOL600c.

### Example 9: Antibody purification development

The purification process used with antisera CF1064 along with the optimised truncated peptide system was developed initially to work on streptavidin columns charged with MOL600 and an absorption column charged with MOL600c. Whilst this process produces effective antibody with good cross reactivity features, further improvements were required to improve consistency and reduce the number of manual steps for automation.

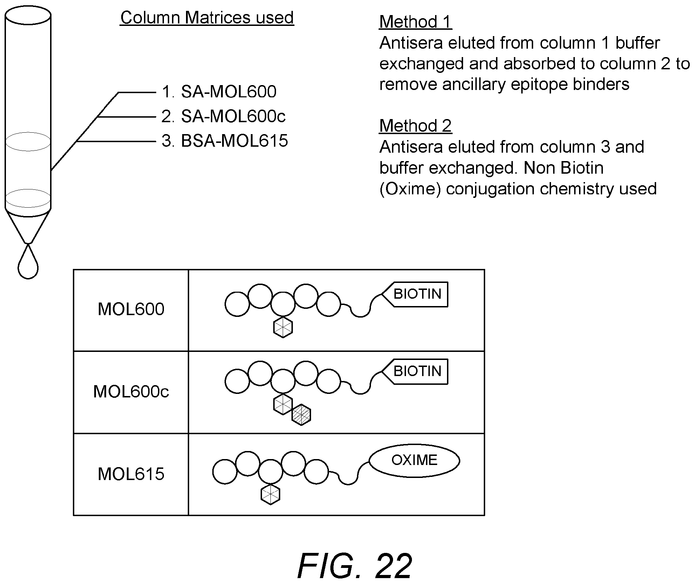

To explore a more refined method using a single pass, other column formats were tried and were shown to be feasible and with potential to be automated (Figure 22). Two different columns types have been used and processes have varied from a single pass method to a post-absorption method where unwanted interactions are mopped up to reduce non-specific binding in the assay. The best option for manufacture is a single pass process and this can be achieved using the peptide MOL615 conjugated to carrier BSA and then derivatised on to an NHS sepharose column.
MOL615: NH₂-Cyc -_{D}Ala-Ser[Gal]-_{D}Ala-Arg-PEG-Oxime

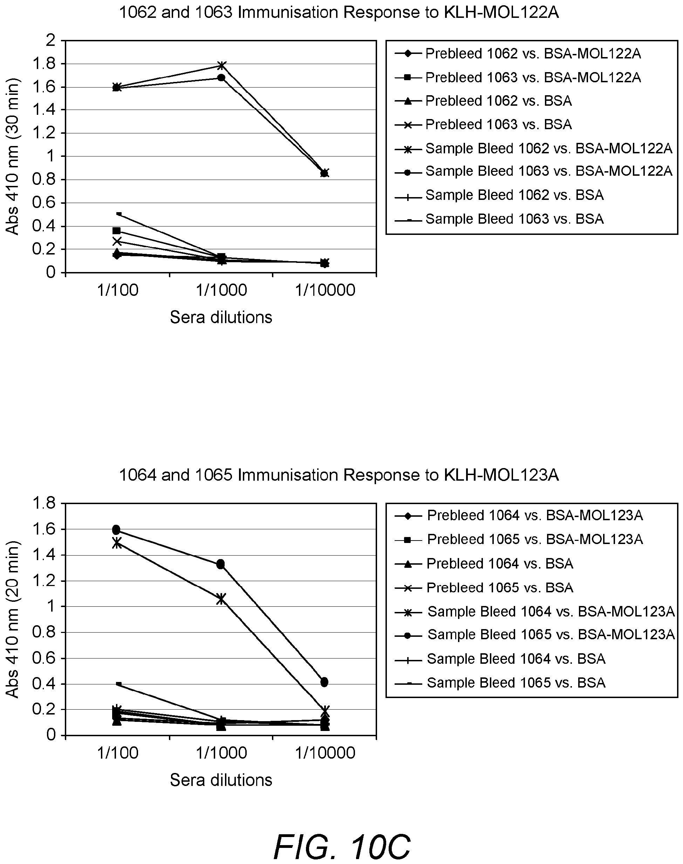

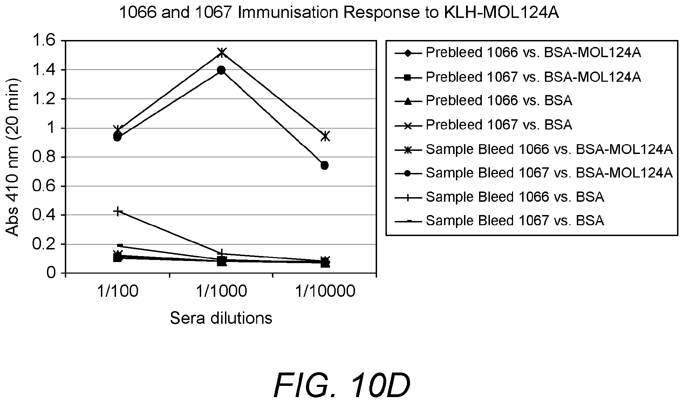

The antibody component in the prototype is formulated as a gold conjugate dried into a conjugate pad within the lateral flow assembly. Conditions have been established for gold conjugate stabilisation and further optimised. The amount of antibody calculated to be required per test is estimated at 85 ng which is sufficient to generate the required validation batches achieve CE Marking. In the long term additional polyclonal reagent will have to be developed and - as more permanent solution - a monoclonal. New immunisations have been started and strong antibody titres have already been identified to the peptide target (Figure 10). Several options will be utilised for monoclonal generation, including commercial hybridoma technology and also in house Phage panning and fab generation (Mologic York and Scotia Biologics).

### Example 10: Sample Buffer

Extraction of sample from the swab, dissolving of peptide and optimal digest conditions for sialidase are the principle requirements of the sample buffer. The following formulation was used:
100mM sodium acetate, 2mM calcium chloride, pH 6.0 containing 0.1% BSA, 0.125% Tween-20 and 0.375% Triton X-100

The buffer has been used successfully to run devices in dry and also extract real samples in spiked recovery.

### Example 11: Lateral flow device

A lateral flow device was constructed per Figure 4.

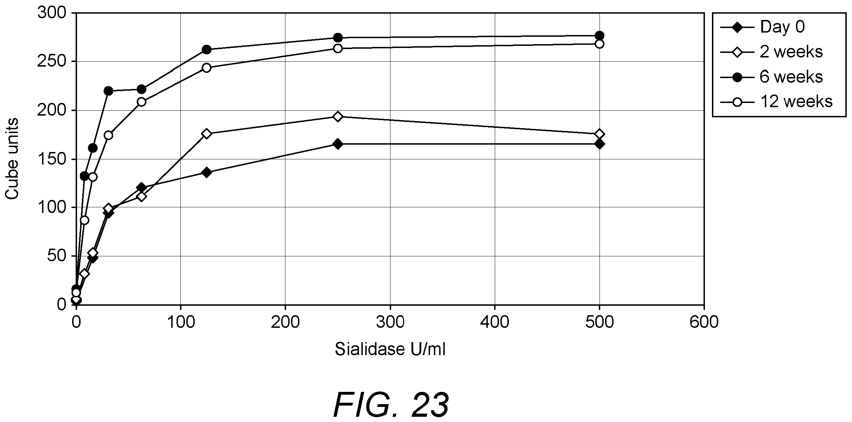

A batch of lateral flow devices were evaluated for stability at 37 ºC over 12 weeks. After 6 weeks, signals enhanced in value over earlier measurements however the shape of the curves and dynamic range appeared relatively consistent (see Figure 23).

### Example 12: Assay optimisation

To satisfy a feasible performance, specification factors such as length of assay time, sensitivity of response to the marker, line signal strength and quality of standard curve were evaluated.

The assay has been shown to work with an incubation time of 5 minutes. This would keep the total time of the assay within 15 minutes from swab sample collection to the reading of the result. In the *in-vitro* diagnostic industry, 15 minutes is generally the norm as an upper limit for a point of care rapid test.

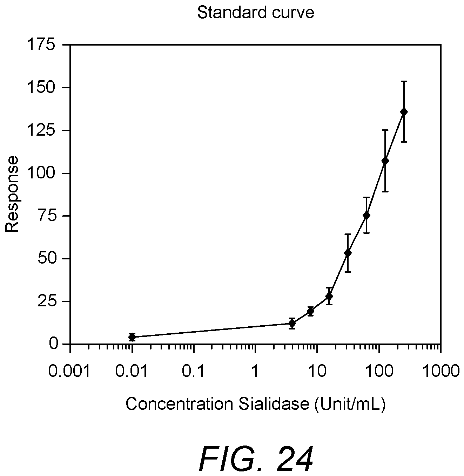

The full relevant range of the marker (sialidase) is believed to be from 4U/ml to 250 U/ml. This is based on the literature by estimating the spread of sample data sets in relation to a fluorescent sialidase reference assay (Marconi et. al., European Journal of Obstetrics and Gynaecology and Reproductive Biology, 2013, vol. 167, pages 205-209). There are assumptions made in this approach as in the BV condition a number of different phenotypes (bacterial sialidases) will be present in the sample as about 20 different bacterial species have been associated with the condition. The assay has been tested across this range of sialidase in buffer and a standard curve representing this range has been achieved in lateral flow (Figure 24).

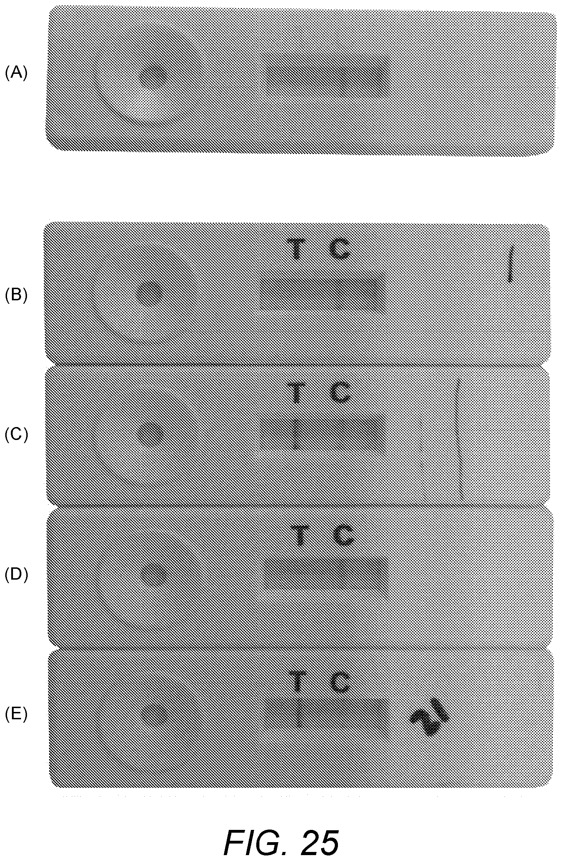

The assay has also been tested on real healthy samples and signal has been recovered by spiking in sialidase. Whether a sample is spun down or not has been shown not to make a difference. The appearance of the test line was not adversely affected by the presence of sample matrix (Figure 25). For the sample to be of sufficient mobility, 0.5 ml of diluent sample buffer was found to be too concentrated as the samples retained too much viscosity. Currently 1 ml is considered the appropriate amount of buffer to use.

### Example 13 Further antibody generation

The following peptide, designated MOL616, was chosen to generate further antibodies: *Dpr(AOA)-dSer-Nva*-Cyc-dAla-Ser(Gal)-dAla-Arg-Phe-dSer-Val-NH₂.

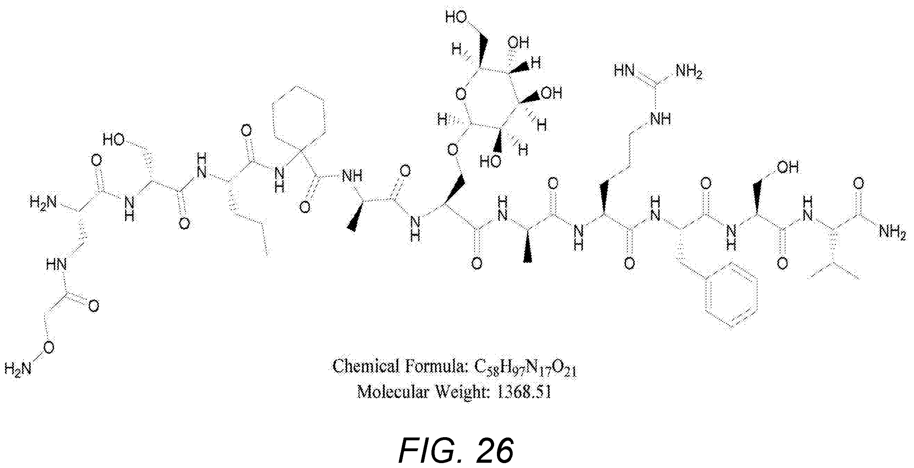

The chemical formula of MOL616 is shown in **Figure 26****.**

The peptide was coupled to KLH, yielding an immunogen denoted KLH-MOL616. Rabbits (Oryctolagus cuniculus) were immunised with KLH-MOL616 and splenocytes were captured and screened with a truncated version of this peptide (MOL615, conjugated to BSA). Counter screens were carried out with the sialylated form of peptide MOL615, MOL615c. The screening process involved a multi-clone screen followed by a subclone screen to yield an antibody specific for the de-sialylated form.

This process led to the generation of a monoclonal antibody denoted antibody 125.1. This antibody is specific for an epitope present in the de-sialidated molecule Cyc-_{D}Ala-Ser[Gal]-_{D}Ala-Arg-PEG-Oxime, more particularly for an epitope present in the de-sialidated peptide Cyc-_{D}Ala-Ser[Gal]-_{D}Ala-Arg.

MOL615 was biotinylated to yield MOL600 (sequence provided in Example 7) and antibody binding affinity and kinetics was measured using bio-inferometry. Briefly, MOL600 or MOL600c was immobilised on a biosensor by streptavidin/biotin interaction. Binding of the antibody was detected by a shift in the reflected light interference.

Using different concentrations of antibody 125.1 (1, 2, 4, 8 or 16 nM respectively) the following binding affinity and kinetics were determined for antibody 125.1:
K_{D} = 7.9 nM; Kₒₙ = 54080M⁻¹s⁻¹; K_{off} = 0.000427 s⁻¹

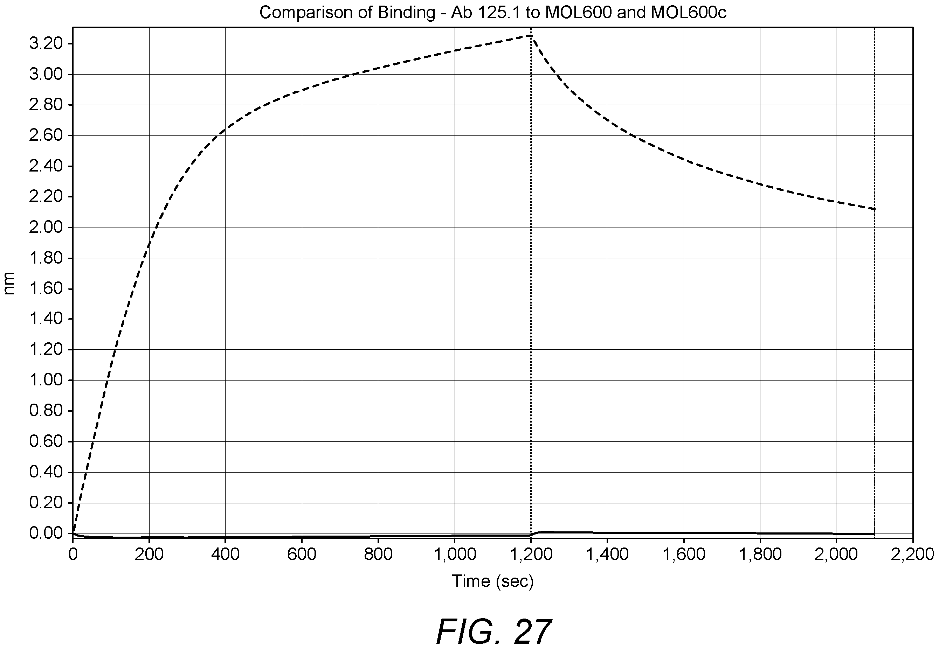

The binding was clearly specific for MOL600 compared to the sialylated form, MOL600c, as shown in Figure 27.

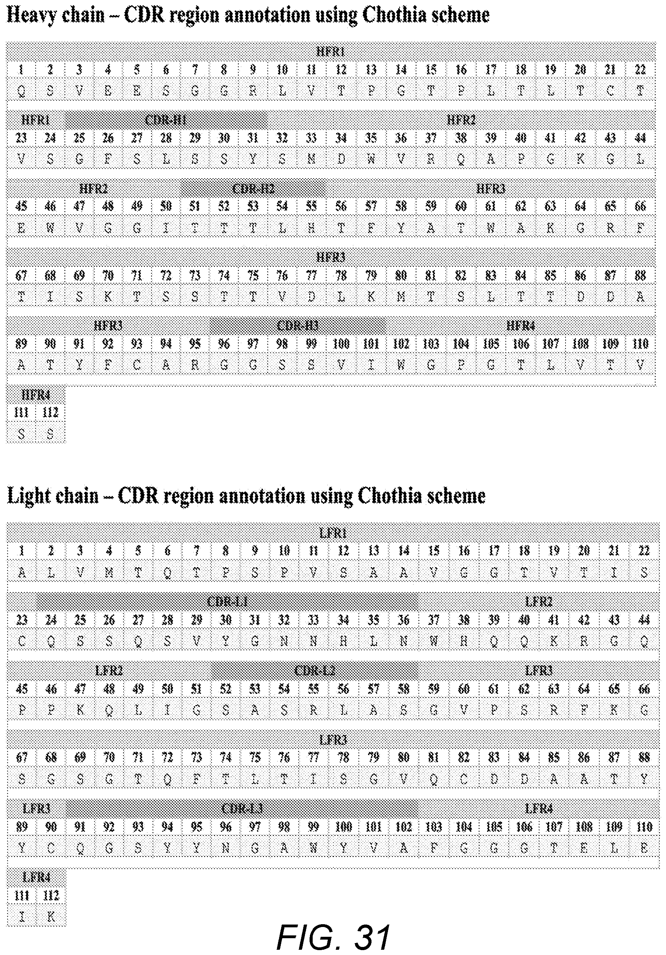

The antibody was sequenced and determined to have a Gamma Heavy chain and a Kappa Light chain. The sequences of this antibody are shown in Figure 31 and listed below.
Antibody-Heavy-Chain 
QSVEESGGRLVTPGTPLTLTCTVSGFSLSSYSMDWVRQAPGKGLEWVGGITTTLHTFYAT
WAKGRFTISKTSSTTVDLKMTSLTTDDAATYFCARGGSSVIWGPGTLVTVSSGQPKAPSV
FPLAPCCGDTPSSTVTLGCLVKGYLPEPVTVTWNSGTLTNGVRTFPSVRQSSGLYSLSSV
VSVTSSSQPVTCNVAHPATNTKVDKTVAPSTCSKPTCPPPELLGGPSVFIFPPKPKDTLMI
SRTPEVTCVVVDVSEDDPEVQFTWYINNEQVRTARPPLREQQFNSTIRVVSTLPIAHEDW
LRGKEFKCKVHNKALPAPIEKTISKARGQPLEPKVYTMGPPREELSSRSVSLTCMINGFYP
SDISVEWEKNGKAEDNYKTTPAVLDSDGSYFLYSKLSVPTSEWQRGDVFTCSVMHEALH
NHYTQKSISRSPG (SEQ ID NO: 7)
Antibody-Light-Chain 
ALVMTQTPSPVSAAVGGTVTISCQSSQSVYGNNHLNWHQQKRGQPPKQLIGSASRLASG
VPSRFKGSGSGTQFTLTISGVQCDDAATYYCQGSYYNGAWYVAFGGGTELEIKRDPVAP
SVLLFPPSKEELTTGTATIVCVANKFYPSDITVTWKVDGTTQQSGIENSKTPQSPEDNTYSL
SSTLSLTSAQYNSHSVYTCEVVQGSASPIVQSFNRGDC
(SEQ ID NO: 8)
Heavy Chain CDR sequences
CDR1 GFSLSSY (SEQ ID NO: 1)
CDR2 TTTLH (SEQ ID NO: 2)
CDR3 GGSSVI (SEQ ID NO: 3)
Light Chain CDR sequences
CDR1 QSSQSVYGNNHLN (SEQ ID NO: 4)
CRD2 SASRLAS (SEQ ID NO: 5)
CDR3 QGSYYNGAWYVA (SEQ ID NO: 6)

### Notable observations:

An additional O-linked glycosylation site was detected in the heavy chain constant region between positions 213 and 216. From the gel image, the form that is glycosylated at this site is similar in abundance to the unglycosylated form.

N-linked glycosylation was detected on heavy chain constant region N@285.

Pyro-Glu(Q) modification observed at N-terminal of heavy chain.

Elimination of C-terminal lysine from regular constant region sequence observed on heavy chain.

### Example 14 Further assay optimisations

For further optimization, two different sialidase activity assays (A and B, see below) were compared. The assay set up was essentially as described in connection with Figure 2. Test antibodies were conjugated to gold and dried onto a carrier, referred to herein as a "conjugate pad". The test antibody in assay A was "1064 purified Mol615 antibody", i.e. a polyclonal antibody purified from serum 1064 using Mol615. The test antibody in assay B was a monoclonal antibody denoted "125.1" (see Example 13). A control antibody was also conjugated to gold and dried onto the conjugate pad. For assay B, this was Chicken IgY, supplied by Lampire, product code 7401403. Capture molecules (polystreptavidin) were immobilised on a nitrocellulose membrane to form a capture line. A separate control capture line was formed. For assay B, this was Anti-Chicken IgY antibodies (supplied by Lampire, product code 7455207). The conjugate pad was laminated onto the nitrocellulose as shown in Figure 2.

The test indicator molecule, NH₂-Cyc-_{D}Ala-Ser[Gal-Sial]-_{D}Ala-Arg-PEG-Biotin (MOL600c), was prepared and 1µg/ml or 3µg/ml was freeze dried onto an inert porous material (a Porex disc) to yield an indicator substrate disc comprising 36ng indicator molecule per disc or 108ng indicator molecule per disc respectively.

Sialidase was tested at the following concentrations: 50, 12.5 and 1.56 U/ml. A negative standard using peptide disk and assay buffer was also run. Responses were measured against cube readings and a visual scale.

The following basic protocol was used:
Ensure all solutions are at room temperature and mixed well.

Test each of the desired conditions with the 50, 12.5 and 1.56U/ml sialidase buffer standards solutions.

Test all standards and the sialidase buffer in triplicate.

Add 1000µl of sialidase standard to an extraction tube, together with an indicator substrate disc. Seal the tube by folding the lid and cap into place. Gently agitate the tube and allow to incubate for 5 minutes with the indicator substrate disc.

Add 4 drops from the extraction tube to the device to the sample pad.

Read at 5 or 10 minutes using the cube reader (Optricon reader from opTricon GmbH (Chembio Diagnostic systems) of Schwarzschildstrasse 1, D-12489 Berlin, Germany) and record test and control line signals.

Features of the test assays are shown below.

| | Assay A | Assay B |
|---|---|---|
| Conjugate Pad | Millipore GFDX | Ahlstrom 8951 |
| Control Line Antibody | Anti-sheep Antibody | Anti-Chicken IgY Antibody |
| Gold conjugate | Test Only | Test and Control |
| Test gold conjugate antibody | 1064 purified Mol615 Polyclonal Antibody | 125.1 Monoclonal Antibody |
| Peptide Concentration | 1µg/ml (36ng per disc) | 3µg/ml (108ng per disc) |
| Device Read Time | 10 minutes | 5 minutes |
| Gold OD | 5 | 4 |
| Sample Pad | MDI FR1 | MDI FR1 |

### (i) Comparison of read times

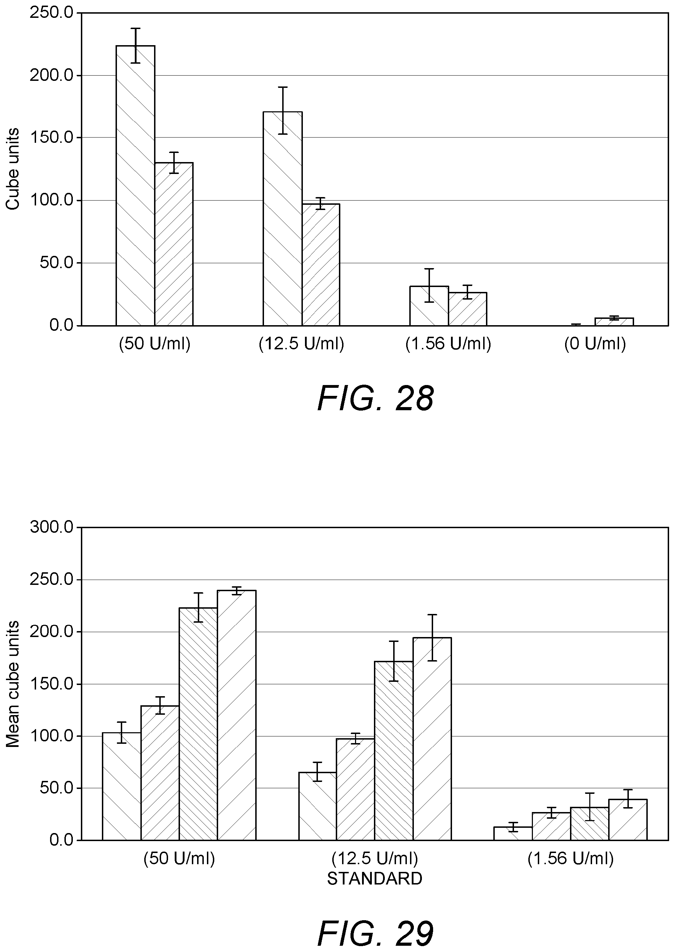

The desirable output is to have a 10-minute test, with a 5-minute incubation and then a 5-minute read time. Results were read at 5 and 10 minutes to investigate the effect of read time on results. Results are shown in Figure 28. As can be seen, assay A requires a 10-minute read time to meet the correct response for the bottom standard where assay B only requires a 5-minute read time. Assay B does not seem to benefit significantly from a 10-minute read time; it meets the desirable output of a 10-minute test with a 5-minute read.

### (ii) comparison of sensitivity

The results of assay B were read after 5 minutes and the results of assay A were read after 10 minutes. Results are shown in Figure 29.

Assay B shows higher cube readings compared to assay A across the 50U/ml, 12.5U/ml and 1.56U/ml standards and has lower cube readings for the 0U/ml standard. Both A and B are giving the correct response for each of the standards and the % CV are within specification. Assay B is giving cube readings for the standards after a shorter device development time and with lower cube readings for the 0 standards. Thus, assay B is superior to assay A.

### (iii) Effect of peptide concentration

Assays A and B were compared to each other and to variants with different peptide concentrations. Assay A' corresponded to assay A, but with a peptide concentration of 3µg/ml (108ng per disc); Assay B' corresponded to assay B, but with a peptide concentration of 1µg/ml (108ng per disc).

The results of assay B and B' were read after 5 minutes and the results of assay A and A' were read after 10 minutes. Results are shown in Figure 30. Higher peptide concentrations gave better results and a peptide concentration of >1µg/ml (>108ng per disc) is preferred.

### Conclusions:

The assay B has improved performance to the assay A in a shorter time because of the changes made to the antibody and the increase of the peptide concentration.

### Example 15 - Additional assay optimisations

### Gold optical density

The effect of increasing the gold conjugate spraying concentration from OD4 to OD6 or OD8 was investigated, and it was found that while OD6 gold gave a slight increase in test line signal compared to OD4, OD8 gave double the test line signal of OD4 at 3.125U/mL sialidase. Thus, using gold conjugation at an OD of about or at least 8 is advantageous.

### Sample/conjugate pad

In a lateral flow assay device, such as the type schematically represented in Figure 2, the sample pad should allow the sample to flow along the device to allow the sample to come into contact with the conjugate pad containing the binding molecules and subsequently come into contact with the capture molecules. Depending on the viscosity of the sample, some sample pad materials may be advantageous.

In order to assess the different sample pads, a synthetic vaginal fluid substitute was prepared using guar gum and bovine mucin (0.25% guar gum, 0.125% mucin, 5% blue latex (Polysciences Inc., 15709, 2.6%, dissolved in water). The synthetic vaginal fluid was allowed to run through all of the sample pads. The time taken for the sample to run the length of the test strip was recorded (see Table below)

| Sample Pad | Time to start of window | Time to run length of window |
|---|---|---|
| 1. FR1 10mm blood separator | 01:20 | 02:20 |
| 2. GF-142 | 01:16 | 02:06 |
| 3. 8964, Ahlstrom | 01:17 | 02:07 |
| 4. 6613, Ahlstrom | 01:45 | 01:20 |
| 5. 6615, Ahlstrom | 02:20 | 01:30 |

Ahlstrom are glass fibre pads of different densities (8964, 6613 and 6615 respectively).

For further comparison, materials were made using two sample pads (FR1 and Ahlstrom 8964). Devices were run using a 4 point standard curve (0, 3.125, 12.5 and 50 U/mL sialidase in aqueous buffer solution), in order to determine whether the alternative sample pad had any effect on either specific or nonspecific signal. Samples run using the 8964 sample pad gave higher specific signal compared to the FR1, with no non-specific binding (NSB).

Using a set of clinical samples spiked with 6.25 U/ml sialidase it was determined that a fibreglass-type pad such as the Ahlstrom 8964 sample pad allows very reliable sample flow and enzyme detection.

### Example 16 - Diagnosis of bacterial vaginosis

Clinical samples were obtained and analysed for criteria such as clue cells to determine a Nugent score, on the basis of which the samples were classed as positive or negative for bacterial vaginosis. The samples were analysed using assay A or assay B (see Example 14).

The results are shown below, wherein se stands for sensitivity; sp stands for specificity; ppv stands for positive predicted value; and nvp stands for negative predicted value.

### Assay A

| | | Nugent | Nugent | |
|---|---|---|---|---|
| | | POS | NEG | TOTAL |
| Assay A | POS | 25 | 0 | 25 |
| Assay A | NEG | 2 | 25 | 27 |
| | TOTAL | 27 | 25 | 52 |

| | se | sp | ppv | npv |
|---|---|---|---|---|
| estimate: | 0.93 | 1 | 1 | 0.93 |
| 95%Cl: | [0.76 ; 0.99] | [0.86 ; 1] | [0.86 ; 1] | [0.76 ; 0.99] |
| p-value: | 5.65E-06 | 5.96E-08 | 5.96E-08 | 5.65E-06 |

### Assay B

| | | NUGENT SCORE | | |
|---|---|---|---|---|
| | | POS | NEG | TOTAL |
| Assay B | POS | 27 | 0 | 27 |
| Assay B | NEG | 0 | 25 | 25 |
| | TOTAL | 27 | 25 | 52 |

| | se | sp | ppv | npv |
|---|---|---|---|---|
| estimate: | 1 | 1 | 1 | 1 |
| 95%Cl: | [0.87 ; 1] | [0.86 ; 1] | [0.87 ; 1] | [0.86 ; 1] |
| p-value: | 1.49E-08 | 5.96E-08 | 1.49E-08 | 5.96E-08 |

Thus, both assays performed well at distinguishing between clinical samples that are positive or negative for bacterial vaginosis. Assay B was superior to assay A.

## Claims

1. An enzyme detection device or enzyme detection kit for detecting the presence in a test sample of cleavage activity of a sialidase enzyme, the device or kit comprising:
(i) an indicator molecule comprising
(a) a peptide comprising the following sequence:
X₁-X₂-X₃[Gal-Sial]-X₄-X₅ (SEQ ID NO: 17)
wherein:
Sial is a sialyl group;
X₃ is an amino acid comprising a glycosyl acceptor group and is selected from Ser, Thr, Tyr, Hyl, Hyp, Asn, Arg or phosphoserine (SEP), preferably wherein X₃ is Ser; and
X₁, X₂, X₄ and X₅ are independently selected from any amino acid provided that at least one, preferably at least two or three, of X₁, X₂, X₄ and X₅ is a D-amino acid and/or a non-standard amino acid or a non-natural amino acid; and
(b) a capture site which remains intact following cleavage of the sialyl group from the indicator molecule by a sialidase enzyme present in the sample;
(ii) a capture zone to receive the test sample, wherein the capture zone comprises capture molecules capable of binding to the capture site of the indicator molecule, irrespective of whether or not the indicator molecule has been cleaved, in order to immobilise the indicator molecule; and
(iii) binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred; and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6.

2. The device or kit of claim 1 wherein at least one of X₁, X₂, X₄ and X₅ is:
(a) Ala, preferably wherein X₁ and X₂ are both Ala, and/or preferably wherein Ala is _{D}Ala or βAla;
(b) a charged amino acid; and/or
(c) a polar amino acid.

3. The device or kit of claim 1 or claim 2, wherein the peptide:
(a) comprises the following sequence:
X₁-X₂-X₃-X₄-X₅-X₆-X₇-X₈-X₉-X₁₀-X₁₁-X₁₂[Gal-Sial]-X₁₃-X₁₄-X₁₅-X₁₆-X₁₇-X₁₈-X₁₉-X₂₀ (SEQ ID NO: 10)
wherein:
Sial is a sialyl group
X₁ is absent or Thr
X₂ is absent or _{D}Ala
X₃ is absent or Nle
X₄ is absent or Glu
X₅ is absent or _{D}Ala
X₆ is absent or Arg
X₇ is absent or selected from Glu, Arg, Ser, Nva, βAla
X₈ is absent or selected from _{D}Ser, _{D}Ala, SEP, Cyc
X₉ is absent or selected from Nva, BIP, _{D}Ala, βAla, Orn
X₁₀ is selected from Cyc, Ser, Ile, _{D}Ala, _{D}Ser
X₁₁ is selected from _{D}Ala, Pro, Orn, Nle
X₁₂ is selected from Ser, Thr, Tyr, Hyl, Hyp, Asn, Arg or SEP, preferably Ser X₁₃ is selected from _{D}Ala, BIP, βAla
X₁₄ is selected from Arg, _{D}Asp, Nle, Orn, Nva
X₁₅ is absent or selected from Phe, BIP, Ser, Glu, _{D}Ala, _{D}Ser X₁₆ is absent or selected from _{D}Ser, Glu
X₁₇ is absent or selected from Val, Ser, Thr
X₁₈ is absent or Cha
X₁₉ is absent or _{D}Ser
X₂₀ is absent or Val;
(b) comprises, or consists of, the following sequence:
(i) Cyc-_{D}Ala-Ser[Gal-Sial]-_{D}Ala-Arg (SEQ ID NO: 11)
(ii) Glu-_{D}Ser-Nva-Cyc-_{D}Ala-Ser[Gal-Sial]- _{D}Ala-Arg-Phe-_{D}Ser-Val (SEQ ID NO: 12)
(iii) Arg-_{D}Ala-BIP-Ser-Pro-Ser[Gal-Sial]-_{D}Ala-_{D}Asp-Ser (SEQ ID NO: 13)
(iv) Ser-SEP-_{D}Ala-Ile-Orn-Ser[Gal-Sial]-_{D}Ala-Nle-Glu (SEQ ID NO: 14)
(v) _{D}Ala-Arg-Nva-_{D}Ser-βAla-_{D}Ala-Nle-Ser[Gal-Sial]-BlP-Orn-_{D}Ala-Glu-Ser (SEQ ID NO: 15); or
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO: 16); and/or
(c) is biased for cleavage by one or more specific sialidases.

4. The device or kit of any one of claims 1-3 wherein:
(a) the binding molecule is specific for the de-sialylated form of the peptide defined in claim 3(b), preferably is specific for an epitope that is present in the peptide motif Cyc-_{D}Ala-Ser[Gal]-_{D}Ala-Arg and that is absent or cryptic in the corresponding sialylated peptide motif Cyc-_{D}Ala-Ser[Gal-Sial]-_{D}Ala-Arg;
(b) the heavy chain of the binding molecule has SEQ ID NO: 7 and/or the light chain of the binding molecule has SEQ ID NO: 8; and/or
(c) the binding molecule is labelled with a reporter molecule.

5. The device or kit of any one of claims 1-4 wherein the capture site of the indicator molecule:
(a) comprises or consists of a biotin molecule or an oxime moiety; and/or is at the N- or C-terminus of the peptide; and/or
(b) is attached to the peptide by a linker.

6. The device or kit of any one of claims 1-5 wherein the indicator molecule:
(a) comprises, or consists of, the following structure:
(i) Cyc-_{D}Ala-Ser[Gal-Sial]-_{D}Ala-Arg-PEG-Biotin
(ii) Biotin-PEG-Asp-Glu-_{D}Ser-Nva-Cyc-_{D}Ala-Ser[Gal-Sial]- _{D}Ala-Arg-Phe-_{D}Ser-Val
(iii) Biotin-PEG-Asp-Arg-_{D}Ala-BIP-Ser-Pro-Ser[Gal-Sial]-_{D}Ala-_{D}Asp-Ser
(iv) Biotin-PEG-Asp-Ser-Ser(PO₃)-_{D}Ala-Ile-Orn-Ser[Gal-Sial]-_{D}Ala-Nle-Glu
(v) Biotin-PEG-Asp-_{D}Ala-Arg-Nva-_{D}Ser-βAla-_{D}Ala-Nle-Ser[Gal-Sial]-BIP-Orn-_{D}Ala-Glu-Ser; or
(vi) Biotin-PEG-Asp-Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val; and/or
(b) comprises (i) a peptide comprising or consisting of the sequence Cyc-_{D}Ala-Ser[Gal-Sial]-_{D}Ala-Arg; and (ii) a capture site that comprises or consists of a biotin molecule or an oxime moiety.

7. An antibody capable of specifically binding to (i) the de-sialylated derivative of a peptide as defined in any one of claims 1-3; or to (ii) an indicator molecule as defined in any one of claims 1-3 or 5-6, wherein the antibody binds preferentially to the de-sialylated derivative over the sialylated peptide or indicator molecule, and wherein the antibody has a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6.

8. An indicator molecule suitable for use in detecting the presence in a test sample of cleavage activity of a sialidase enzyme, the indicator molecule comprising:
a) a peptide as defined in any one of claims 1-3; and
b) a capture site which remains intact following cleavage of the sialyl group from the indicator molecule by a sialidase enzyme present in the sample, wherein the indicator molecule is preferably as defined in any one of claims 1-3 or 5-6.

9. A peptide comprising or consisting of the following sequence:
(i) Cyc-_{D}Ala-Ser[Gal-Sial]-_{D}Ala-Arg (SEQ ID NO: 11)
(ii) Glu-_{D}Ser-Nva-Cyc-_{D}Ala-Ser[Gal-Sial]- _{D}Ala-Arg-Phe-_{D}Ser-Val (SEQ ID NO: 12)
(iii) Arg-_{D}Ala-BIP-Ser-Pro-Ser[Gal-Sial]-_{D}Ala-_{D}Asp-Ser (SEQ ID NO: 13)
(iv) Ser-SEP-_{D}Ala-Ile-Orn-Ser[Gal-Sial]-_{D}Ala-Nle-Glu (SEQ ID NO: 14)
(v) _{D}Ala-Arg-Nva-_{D}Ser-βAla-_{D}Ala-Nle-Ser[Gal-Sial]-BIP-Orn-_{D}Ala-Glu-Ser (SEQ ID NO: 15); or
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO: 16).

10. A peptide for use in generating an antibody according to claim 7, wherein the peptide is a de-sialylated derivative of the peptide as defined in any one of claims 1-3.

11. A method for detecting the presence or absence in a test sample of cleavage activity of a sialidase enzyme, the method comprising:
(i) bringing an indicator molecule as defined in any one of claims 1-3 or 5-6 into contact with the test sample;
(ii) adding to the test sample binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6;
(iii) capturing the de-sialylated derivative of the indicator molecule at a capture zone through binding of capture molecules in the capture zone to the capture site, said capture molecules being able to bind to the capture site irrespective of whether or not the indicator molecule has been cleaved; and
(iv) detecting cleavage of the sialyl group from the indicator molecule by determining binding of the binding molecules to the de-sialylated derivative of the indicator molecule captured in the capture zone.

12. A method for detecting the presence or absence in a test sample of cleavage activity of a sialidase enzyme, the method comprising:
(i) adding the test sample to a capture zone comprising capture molecules, said capture molecules being bound to the capture site of an indicator molecule as defined in any one of claims 1-3 or 5-6 wherein said capture molecules remain bound to the capture site irrespective of whether or not cleavage of the sialyl group from the indicator molecule by sialidase enzyme present in the sample occurs;
(ii) adding binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6;
(iii) detecting cleavage of the sialyl group from the indicator molecule by determining binding of the binding molecules to the de-sialylated derivative of the indicator molecule captured in the capture zone.

13. A method for diagnosing bacterial vaginosis in a test sample by detecting cleavage activity of a sialidase enzyme in the sample, the method comprising:
(i) bringing an indicator molecule as defined in any one of claims 1-3 or 5-6 into contact with the test sample;
(ii) adding to the test sample binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6;
(iii) capturing the de-sialylated derivative of the indicator molecule at a capture zone through binding of capture molecules in the capture zone to the capture site, said capture molecules being able to bind to the capture site irrespective of whether or not the indicator molecule has been cleaved; and
(iv) detecting cleavage of the sialyl group from the indicator molecule by determining binding of the binding molecules to the de-sialylated derivative of the indicator molecule captured in the capture zone wherein an increased level of cleavage compared to a control diagnoses bacterial vaginosis.

14. An enzyme detection kit for detecting the presence in a test sample of cleavage activity of a sialidase enzyme, the kit comprising:
(i) an indicator molecule as defined in any one of claims 1-3 wherein the indicator molecule is preferably freeze-dried onto a carrier;
(ii) capture molecules capable of binding to the capture site of the indicator molecule, irrespective of whether or not the indicator molecule has been cleaved, wherein the capture molecules preferably comprise streptavidin;
(iii) a solid support to which the capture molecules can be attached, or are attached, to form a capture zone to receive the test sample, wherein the solid support preferably comprises nitrocellulose; and
(iv) binding molecules capable of binding to the de-sialylated derivative of the indicator molecule, wherein the binding molecules are incapable of binding to the indicator molecule unless and until cleavage of the sialyl group from the indicator molecule by sialidase enzyme present in the sample has occurred and wherein the binding molecules are antibodies having a heavy chain with 3 CDRs and a light chain with 3 CDRs, wherein the heavy chain CDR1 has SEQ ID NO:1; the heavy chain CDR2 has SEQ ID NO:2; the heavy chain CDR3 has SEQ ID NO:3; the light chain CDR1 has SEQ ID NO: 4; the light chain CDR2 has SEQ ID NO: 5; and the light chain CDR3 has SEQ ID NO: 6.

15. Use of an enzyme detection device or enzyme detection kit according to any one of claims 1-6, method according to any one of claims 11-13 or the enzyme detection kit according to claim 14 for diagnosing bacterial vaginosis in a test sample.

## Patentansprüche

1. Enzym-Nachweisvorrichtung oder Enzym-Nachweiskit zum Nachweis des Vorhandenseins einer Spaltungsaktivität eines Sialidase-Enzyms in einer Testprobe, wobei die Vorrichtung oder der Kit umfasst:
(i) ein Indikatormolekül, umfassend:
(a) ein Peptid mit der folgenden Sequenz:
X₁-X₂-X₃ [Gal-Sial]-X₄- X₅ (SEQ ID NO: 17)
wobei:
Sial eine Sialylgruppe ist;
X₃ eine Aminosäure ist, die eine Glykosyl-Akzeptorgruppe umfasst und aus Ser, Thr, Tyr, Hyl, Hyp, Asn, Arg oder Phosphoserin (SEP) ausgewählt wird, wobei X₃ vorzugsweise Ser ist; und
X₁, X₂, X₄ und X₅ unabhängig voneinander aus einer beliebigen Aminosäure ausgewählt werden, vorausgesetzt, dass mindestens eine, vorzugsweise mindestens zwei oder drei, von X₁, X₂, X₄ und X₅ eine D-Aminosäure und/oder eine Nicht-Standard-Aminosäure oder eine nicht-natürliche Aminosäure ist; und
(b) eine Aufnahmestelle, die nach Abspaltung der Sialylgruppe vom Indikatormolekül durch ein in der Probe vorhandenes Sialidase-Enzym intakt bleibt;
(ii) eine Aufnahmezone zur Aufnahme der Testprobe, wobei die Aufnahmezone Fängermoleküle umfasst, die in der Lage sind, an die Aufnahmestelle des Indikatormoleküls zu binden, unabhängig davon, ob das Indikatormolekül gespalten worden ist oder nicht, um das Indikatormolekül zu immobilisieren; und
(iii) Bindungsmoleküle, die in der Lage sind, an das desialylierte Derivat des Indikatormoleküls zu binden, wobei die Bindungsmoleküle nicht in der Lage sind, an das Indikatormolekül zu binden, bis eine Abspaltung der Sialylgruppe von dem Indikatormolekül durch ein in der Probe vorhandenes Sialidase-Enzym stattgefunden hat; und wobei die Bindungsmoleküle Antikörper sind, die eine schwere Kette mit 3 CDRs und eine leichte Kette mit 3 CDRs aufweisen, wobei die schwere Kette CDR1 die SEQ ID NO: 1 hat; die schwere Kette CDR2 die SEQ ID NO: 2 hat; die schwere Kette CDR3 die SEQ ID NO: 3 hat; die leichte Kette CDR1 die SEQ ID NO: 4 hat; die leichte Kette CDR2 die SEQ ID NO: 5 hat; und die leichte Kette CDR3 die SEQ ID NO: 6 hat.

2. Vorrichtung oder Kit nach Anspruch 1, wobei mindestens eines von X₁, X₂, X₄ und X₅ Folgendes ist:
(a) Ala, wobei X₁ und X₂ vorzugsweise beide Ala sind, und/oder wobei Ala vorzugsweise _{D}Ala oder βAla ist;
(b) eine geladene Aminosäure; und/oder
(c) eine polare Aminosäure.

3. Vorrichtung oder Kit nach Anspruch 1 oder Anspruch 2, wobei das Peptid:
(a) die folgende Sequenz umfasst:
X₁-X₂-X₃-X₄-X₅-X₆-X₇-X_{g}-X₉-X₁₀-X₁₁-X₁₂[Gal-Sial] X₁₃-X₁₄-X₁₅-X₁₆-X₁₇-X₁₈-X₁₉-X₂₀ (SEQ ID NO: 10),
wobei:
Sial eine Sialylgruppe ist;
X₁ nicht vorhanden oder Thr ist;
X₂ nicht vorhanden oder _{D}Aia ist;
X₃ nicht vorhanden oder Nle ist;
X₄ nicht vorhanden oder Glu ist;
X₅ nicht vorhanden oder _{D}Ala ist;
X₆ nicht vorhanden oder Arg ist;
X₇ nicht vorhanden ist oder ausgewählt ist aus Glu, Arg, Ser, Nva, _{β}Ala;
X₈ nicht vorhanden ist oder ausgewählt ist aus _{D}Ser, _{D}Ala, SEP, Cyc;
X₉ nicht vorhanden ist oder ausgewählt ist aus Nva, BIP, _{D}Ala, βAla, Orn;
X₁₀ ausgewählt ist aus Cyc, Ser, Ile, _{D}Ala, _{D}Ser;
X₁₁ ausgewählt ist aus _{D}Ala, Pro, Orn, Nle;
X₁₂ ausgewählt ist aus Ser, Thr, Tyr, Hyl, Hyp, Asn, Arg oder SEP, vorzugsweise Ser;
X₁₃ ausgewählt ist aus _{D}Ala, BIP, _{β}Ala;
X₁₄ ausgewählt ist aus Arg, _{D}Asp, Nle, Orn, Nva;
X₁₅ nicht vorhanden ist oder ausgewählt ist aus Phe, BIP, Ser, Glu, _{D}Ala, _{D}Ser;
X₁₆ nicht vorhanden ist oder ausgewählt ist aus _{D}Ser, Glu;
X₁₇ nicht vorhanden ist oder ausgewählt ist aus Val, Ser, Thr;
X₁₈ nicht vorhanden oder Cha ist;
X₁₉ nicht vorhanden oder _{D}Ser ist;
X₂₀ nicht vorhanden oder Val ist;
(b) die folgende Sequenz umfasst oder daraus besteht:
(i) Cyc-_{D}Ala-Ser[Gal-Sial]-_{D}Ala-Arg (SEQ ID NO: 11)
(ii) Glu-_{D}Ser-Nva-Cyc-_{D}Ala-Ser[Gal-Sial]-_{D}Ala-Arg-Phe-_{D}Ser-Val (SEQ ID NO: 12)
(iii) Arg-_{D}Ala-BIP-Ser-Pro-Ser[Gal-Sial]-_{D}Ala-_{D}Asp-Ser (SEQ ID NO: 13)
(iv) Ser-SEP-_{D}Ala-Ile-Orn-Ser[Gal-Sial]-_{D}Ala-Nle-Glu (SEQ ID NO: 14)
(v) _{D}Ala-Arg-Nva-_{D}Ser-βAla-_{D}Ala-Nle-Ser[Gal-Sial]-BIP-Orn-_{D}Ala-Glu-Ser (SEQ ID NO: 15); oder
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO: 16); und/oder
(c) für die Spaltung durch eine oder mehrere spezifische Sialidasen vorgespannt ist.

4. Vorrichtung oder das Kit nach einem der Ansprüche 1-3, wobei:
(a) das Bindungsmolekül spezifisch für die desialylierte Form des in Anspruch 3(b) definierten
Peptids ist, vorzugsweise spezifisch für ein Epitop ist, das in dem Peptidmotiv Cyc-_{D}Ala-Ser[Gal]-_{D}Ala-Arg vorhanden ist und das in dem entsprechenden sialylierten Peptidmotiv Cyc-_{D}Ala- Ser[Gal-Sial]-_{D}Ala-Arg fehlt oder kryptisch ist;
(b) die schwere Kette des Bindungsmoleküls die SEQ ID NO: 7 hat und/oder die leichte Kette des Bindungsmoleküls die SEQ ID NO: 8 hat; und/oder
(c) das Bindungsmolekül mit einem Reportermolekül markiert ist.

5. Vorrichtung oder Kit nach einem der Ansprüche 1-4, wobei die Aufnahmestelle des Indikatormoleküls:
(a) ein Biotin-Molekül oder einen Oxim-Anteil enthält oder daraus besteht; und/oder sich am N- oder C-Terminus des Peptids befindet; und/oder
(b) über einen Linker an das Peptid gebunden ist.

6. Vorrichtung oder Kit nach einem der Ansprüche 1-5, wobei das Indikatormolekül:
(a) die folgende Struktur umfasst oder besteht aus:
(i) Cyc-_{D}Ala-Ser[Gal-Sial]-_{D}Ala-Arg-PEG-Biotin
(ii) Biotin-PEG-Asp-Glu-_{D}Ser-Nva-Cyc-_{D}Ala-Ser[Gal-Sial]-_{D}Ala-Arg-Phe-_{D}Ser-Val
(iii) Biotin-PEG-Asp-Arg-_{D}Ala-BIP-Ser-Pro-Ser[Gal-Sial]-_{D}Ala-_{D}Asp-Ser
(iv) Biotin-PEG-Asp-Ser-Ser(PO₃)-_{D}Ala-Ile-Orn-Ser[Gal-Sial]-_{D}Ala-Nle-Glu
(v) Biotin-PEG-Asp-_{D}Ala-Arg-Nva-_{D}Ser-βAla-_{D}Ala-Nle-Ser[Gal-Sial]-BIP-Orn-_{D}Ala-Glu-Ser; oder
(vi) Biotin-PEG-Asp-Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val; und/oder
(b) (i) ein Peptid umfasst, das die Sequenz Cyc-_{D}Ala-Ser[Gal-Sial]-_{D}Ala-Arg umfasst oder daraus besteht; und (ii) eine Aufnahmestelle umfasst, die ein Biotin-Molekül oder einen Oxim-Anteil umfasst oder daraus besteht.

7. Antikörper, der in der Lage ist, spezifisch an (i) das desialylierte Derivat eines Peptids gemäß Definition in einem der Ansprüche 1-3 zu binden; oder spezifisch an (ii) ein Indikatormolekül gemäß Definition in einem der Ansprüche 1-3 oder 5-6 zu binden, wobei der Antikörper vorzugsweise an das desialylierte Derivat gegenüber dem sialylierten Peptid oder Indikatormolekül bindet, und wobei der Antikörper eine schwere Kette mit 3 CDRs und eine leichte Kette mit 3 CDRs aufweist, wobei die schwere Kette CDR1 die SEQ ID NO:1 hat; die schwere Kette CDR2 die SEQ ID NO:2 hat; die schwere Kette CDR3 die SEQ ID NO: 3 hat; die leichte Kette CDR1 die SEQ ID NO: 4 hat; die leichte Kette CDR2 hat SEQ ID NO: 5 hat; und die leichte Kette CDR3 die SEQ ID NO: 6 hat.

8. Indikatormolekül, das zur Verwendung beim Nachweis des Vorhandenseins einer Spaltungsaktivität eines Sialidase-Enzyms in einer Testprobe geeignet ist, wobei das Indikatormolekül umfasst:
(a) ein Peptid gemäß Definition in einem der Ansprüche 1-3; und
(b) eine Aufnahmestelle, die nach Abspaltung der Sialylgruppe von dem Indikatormolekül durch ein in der Probe vorhandenes Sialidase-Enzym intakt bleibt, wobei das Indikatormolekül vorzugsweise wie in einem der Ansprüche 1-3 oder 5-6 definiert ist.

9. Peptid, das die folgende Sequenz umfasst oder aus ihr besteht:
(i) Cyc-_{D}Ala-Ser[Gal-Sial]-_{D}Ala-Arg (SEQ ID NO: 11)
(ii) Glu-_{D}Ser-Nva-Cyc-_{D}Ala-Ser[Gal-Sial]-_{D}Ala-Arg-Phe-_{D}Ser-Val (SEQ ID NO: 12)
(iii) Arg-_{D}Ala-BIP-Ser-Pro-Ser[Gal-Sial]-_{D}Ala-_{D}Asp-Ser (SEQ ID NO: 13)
(iv) Ser-SEP-_{D}Ala-Ile-Orn-Ser[Gal-Sial]-_{D}Ala-Nle-Glu (SEQ ID NO: 14)
(v) _{D}Ala-Arg-Nva-_{D}Ser-βAla-_{D}Ala-Nle-Ser[Gal-Sial]-BIP-Orn-_{D}Ala-Glu-Ser (SEQ ID NO: 15); oder
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO: 16).

10. Peptid zur Verwendung bei der Herstellung eines Antikörpers nach Anspruch 7, wobei das Peptid ein desialyliertes Derivat des Peptids gemäß Definition in einem der Ansprüche 1 bis 3 ist.

11. Verfahren zum Nachweis des Vorhandenseins oder der Abwesenheit einer Spaltungsaktivität eines Sialidase-Enzyms in einer Testprobe, wobei das Verfahren umfasst:
(i) Inkontaktbringen eines Indikatormoleküls gemäß Definition in einem der Ansprüche 1-3 oder 5-6 mit der Testprobe;
(ii) Zugeben von Bindungsmolekülen zu der Testprobe, die in der Lage sind, an das desialylierte Derivat des Indikatormoleküls zu binden, wobei die Bindungsmoleküle nicht in der Lage sind, an das Indikatormolekül zu binden, bis die Abspaltung der Sialylgruppe von dem Indikatormolekül durch das in der Probe vorhandene Sialidase-Enzym stattgefunden hat, und wobei die Bindungsmoleküle Antikörper mit einer schweren Kette mit 3 CDRs und einer leichten Kette mit 3 CDRs sind, wobei die schwere Kette CDR1 die SEQ ID NO: 1 hat; die schwere Kette CDR2 die SEQ ID NO: 2 hat; die schwere Kette CDR3 die SEQ ID NO: 3 hat; die leichte Kette CDR1 die SEQ ID NO: 4 hat; die leichte Kette CDR2 die SEQ ID NO: 5 hat; und die leichte Kette CDR3 die SEQ ID NO: 6 hat;
(iii) Aufnehmen des desialylierten Derivats des Indikatormoleküls an einer Aufnahmezone durch Bindung von Fängermolekülen in der Aufnahmezone an die Aufnahmestelle, wobei die Fängermoleküle in der Lage sind, an die Aufnahmestelle zu binden, unabhängig davon, ob das Indikatormolekül gespalten wurde oder nicht; und
(iv) Nachweis einer Abspaltung der Sialylgruppe von dem Indikatormolekül durch Bestimmung der Bindung der Bindungsmoleküle an das desialylierte Derivat des Indikatormoleküls, das in der Aufnahmezone aufgenommen wurde.

12. Verfahren zum Nachweis des Vorhandenseins oder der Abwesenheit einer Spaltungsaktivität eines Sialidase-Enzyms in einer Testprobe, wobei das Verfahren umfasst:
(i) Zugeben der Testprobe zu einer Aufnahmezone, die Fängermoleküle umfasst, wobei die Fängermoleküle an die Aufnahmestelle eines Indikatormoleküls, wie in einem der Ansprüche 1-3 oder 5-6 definiert, gebunden sind, wobei die Fängermoleküle an die Aufnahmestelle gebunden bleiben, unabhängig davon, ob eine Abspaltung der Sialylgruppe von dem Indikatormolekül durch das in der Probe vorhandene Sialidase-Enzym stattfindet oder nicht;
(ii) Hinzufügen von Bindungsmolekülen, die in der Lage sind, an das desialylierte Derivat des Indikatormoleküls zu binden, wobei die Bindungsmoleküle nicht in der Lage sind, an das Indikatormolekül zu binden, bis eine Abspaltung der Sialylgruppe von dem Indikatormolekül durch das in der Probe vorhandene Sialidase-Enzym stattgefunden hat, und wobei die Bindungsmoleküle Antikörper mit einer schweren Kette mit 3 CDRs und einer leichten Kette mit 3 CDRs sind, wobei die schwere Kette CDR1 die SEQ ID NO: 1 hat; die schwere Kette CDR2 die SEQ ID NO: 2 hat; die schwere Kette CDR3 die SEQ ID NO: 3 hat; die leichte Kette CDR1 die SEQ ID NO: 4 hat; die leichte Kette CDR2 die SEQ ID NO: 5 hat; und die leichte Kette CDR3 die SEQ ID NO: 6 hat;
(iii) Nachweisen einer Abspaltung der Sialylgruppe von dem Indikatormolekül durch Bestimmen, dass die Bindungsmoleküle an das desialylierte Derivat des Indikatormoleküls gebunden sind, das in der Aufnahmezone aufgenommen wurde.

13. Verfahren zur Diagnose von bakterieller Vaginose in einer Testprobe durch Nachweisen einer Spaltungsaktivität eines Sialidase-Enzyms in der Probe, wobei das Verfahren umfasst:
(i) Inkontaktbringen eines Indikatormoleküls gemäß Definition in einem der Ansprüche 1-3 oder 5-6 mit der Testprobe;
(ii) Hinzufügen von Bindungsmolekülen zu der Testprobe, die in der Lage sind, sich an das desialylierte Derivat des Indikatormoleküls zu binden, wobei die Bindungsmoleküle nicht in der Lage sind, sich an das Indikatormolekül zu binden, bis eine Abspaltung der Sialylgruppe von dem Indikatormolekül durch ein in der Probe vorhandenes Sialidase-Enzym stattgefunden hat, und wobei die Bindungsmoleküle Antikörper sind, die eine schwere Kette mit 3 CDRs und eine leichte Kette mit 3 CDRs aufweisen, wobei die schwere Kette CDR1 die SEQ ID NO: 1 hat; die schwere Kette CDR2 die SEQ ID NO: 2 hat; die schwere Kette CDR3 die SEQ ID NO: 3 hat; die leichte Kette CDR1 die SEQ ID NO: 4 hat; die leichte Kette CDR2 die SEQ ID NO: 5 hat; und die leichte Kette CDR3 die SEQ ID NO: 6 hat;
(iii) Aufnehmen des desialylierten Derivats des Indikatormoleküls an einer Aufnahmezone durch Bindung von Fängermolekülen in der Aufnahmezone an die Aufnahmestelle, wobei die Fängermoleküle in der Lage sind, an die Aufnahmestelle zu binden, unabhängig davon, ob das Indikatormolekül gespalten wurde oder nicht; und
(iv) Nachweisen einer Abspaltung der Sialylgruppe von dem Indikatormolekül durch Bestimmen der Bindung der Bindungsmoleküle an das desialylierte Derivat des Indikatormoleküls, das in der Aufnahmezone aufgenommen wurde, wobei ein erhöhter Grad der Abspaltung im Vergleich zu einer Kontrolle bakterielle Vaginose diagnostiziert.

14. Enzym-Nachweis-Kit zum Nachweis des Vorhandenseins einer Spaltungsaktivität eines Sialidase-Enzyms in einer Testprobe, wobei der Kit umfasst:
(i) ein Indikatormolekül gemäß Definition in einem der Ansprüche 1 bis 3, wobei das Indikatormolekül vorzugsweise auf einen Träger gefriergetrocknet wird;
(ii) Fängermoleküle, die in der Lage sind, an die Aufnahmestelle des Indikatormoleküls zu binden, unabhängig davon, ob das Indikatormolekül gespalten worden ist oder nicht, wobei die Fängermoleküle vorzugsweise Streptavidin umfassen;
(iii) einen festen Träger, an den die Fängermoleküle angehängt werden können oder angehängt werden, um eine Aufnahmezone zur Aufnahme der Testprobe zu bilden, wobei der feste Träger vorzugsweise Nitrocellulose umfasst; und
(iv) Bindungsmoleküle, die in der Lage sind, an das desialylierte Derivat des Indikatormoleküls zu binden, wobei die Bindungsmoleküle nicht in der Lage sind, an das Indikatormolekül zu binden, bis eine Abspaltung der Sialylgruppe von dem Indikatormolekül durch das in der Probe vorhandene Sialidase-Enzym stattgefunden hat, und wobei die Bindungsmoleküle Antikörper sind, die eine schwere Kette mit 3 CDRs und eine leichte Kette mit 3 CDRs aufweisen, wobei die schwere Kette CDR1 die SEQ ID NO: 1 hat; die schwere Kette CDR2 die SEQ ID NO: 2 hat; die schwere Kette CDR3 die SEQ ID NO: 3 hat; die leichte Kette CDR1 die SEQ ID NO: 4 hat; die leichte Kette CDR2 die SEQ ID NO: 5 hat; und die leichte Kette CDR3 die SEQ ID NO: 6 hat.

15. Verwendung einer Enzym-Nachweisvorrichtung oder eines Enzym-Nachweiskits nach einem der Ansprüche 1 bis 6, eines Verfahrens nach einem der Ansprüche 11 bis 13 oder des Enzym-Nachweiskits nach Anspruch 14 zur Diagnose von bakterieller Vaginose in einer Testprobe.

## Revendications

1. Dispositif de détection d'enzymes ou kit de détection d'enzymes permettant de détecter la présence dans un échantillon à tester d'une activité de clivage d'une enzyme sialidase, le dispositif ou le kit comprenant :
(i) une molécule indicatrice comprenant
(a) un peptide comprenant la séquence suivante :
X₁-X₂-X₃[Gal-Sial]-X₄-X₅ (SEQ ID NO : 17)
dans lequel :
Sial est un groupe sialyle ;
X₃ est un acide aminé comprenant un groupe accepteur de glycosyle et est choisi parmi Ser, Thr, Tyr, Hyl, Hyp, Asn, Arg ou la phosphosérine (SEP), de préférence dans lequel X₃ est Ser ; et
X₁, X₂, X₄ et X₅ sont indépendamment choisis parmi un quelconque acide aminé à condition qu'au moins un, de préférence au moins deux ou trois, parmi X₁, X₂, X₄ et X₅ soit un acide aminé D et/ou un acide aminé non standard ou un acide aminé non naturel ; et
(b) un site de capture qui reste intact après le clivage du groupe sialyle de la molécule indicatrice par une enzyme sialidase présente dans l'échantillon ;
(ii) une zone de capture pour recevoir l'échantillon à tester, dans lequel la zone de capture comprend des molécules de capture capables de se lier au site de capture de la molécule indicatrice, indépendamment du fait que la molécule indicatrice a été clivée ou non, afin d'immobiliser la molécule indicatrice ; et
(iii) des molécules de liaison capables de se lier au dérivé désialylé de la molécule indicatrice, dans lequel les molécules de liaison sont incapables de se lier à la molécule indicatrice jusqu'à ce que le clivage du groupe sialyle de la molécule indicatrice par une enzyme sialidase présente dans l'échantillon se soit produit ; et dans lequel les molécules de liaison sont des anticorps ayant une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6.

2. Dispositif ou kit selon la revendication 1 dans lequel au moins un parmi X₁, X₂, X₄ et X₅ est :
(a) Ala, de préférence dans lequel X₁ et X₂ sont tous deux Ala, et/ou de préférence dans lequel Ala est _{D}Ala ou _{β}Ala ;
(b) un acide aminé chargé ; et/ou
(c) un acide aminé polaire.

3. Dispositif ou kit selon la revendication 1 ou la revendication 2, dans lequel le peptide :
(a) comprend la séquence suivante :
X₁-X₂-X-₃-X₄-X₅-X₆-X₇-X₈-X₉-X₁₀-X₁₁-X₁₂[Gal-Sial]-X₁₃-X₁₄-X₁₅-X₁₆-X₁₇-X₁₈-X₁₉-X₂₀ (SEQ ID NO : 10)
dans lequel :
Sial est un groupe sialyle
X₁ est absent ou Thr
X₂ est absent ou _{D}Ala
X₃ est absent ou Nle
X₄ est absent ou Glu
X₅ est absent ou _{D}Ala
X₆ est absent ou Arg
X₇ est absent ou choisi parmi Glu, Arg, Ser, Nva, βAla
X₈ est absent ou choisi parmi _{D}Ser, _{D}Ala, SEP, Cyc
X₉ est absent ou choisi parmi Nva, BIP, _{D}Ala, βAla, Orn
X₁₀ est choisi parmi Cyc, Ser, Ile, _{D}Ala, _{D}Ser
X₁₁ est choisi parmi _{D}Ala, Pro, Orn, Nle
X₁₂ est choisi parmi Ser, Thr, Tyr, Hyl, Hyp, Asn, Arg ou SEP, de préférence Ser
X₁₃ est choisi parmi _{D}Ala, BIP, βAla
X₁₄ est choisi parmi Arg, _{D}Ala, Nle, Orn, Nva
X₁₅ est absent ou choisi parmi Phe, BIP, Ser, Glu, _{D}Ala, _{D}Ser
X₁₆ est absent ou choisi parmi _{D}Ser, Glu
X₁₇ est absent ou choisi parmi Val, Ser, Thr
X₁₈ est absent ou Cha
X₁₉ est absent ou _{D}Ser
X₂₀ est absent ou Val ;
(b) comprend, ou se compose de, la séquence suivante :
(i) Cyc-_{D}Ala-Ser[Gal-Sial]-_{D}Ala-Arg (SEQ ID NO : 11)
(ii) Glu-_{D}Ser-Nva-Cyc-_{D}Ala-Ser[Gal-Sial]-_{D}Ala-Arg-Phe-_{D}Ser-Val (SEQ ID NO : 12)
(iii) Arg-_{D}Ala-BIP-Ser-Pro-Ser[Gal-Sial]-_{D}Ala-_{D}Asp-Ser (SEQ ID NO : 13)
(iv) Ser-SEP-_{D}Ala-Ile-Orn-Ser[Gal-Sial]-_{D}Ala-Nle-Glu (SEQ ID NO : 14)
(v) _{D}Ala-Arg-Nva-_{D}Ser-βAla-_{D}Ala-Nle-Ser[Gal-Sial]-BIP-Orn-_{D}Ala-Glu-Ser (SEQ ID NO : 15) ; ou
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO : 16) ; et/ou
(c) est biaisée pour un clivage par une ou plusieurs sialidases spécifiques.

4. Dispositif ou kit selon l'une quelconque des revendications 1 à 3, dans lequel :
(a) la molécule de liaison est spécifique de la forme désialylée du peptide défini dans la revendication 3(b), est de préférence spécifique d'un épitope qui est présent dans le motif peptidique Cyc-_{D}Ala-Ser[Gal]-_{D}Ala-Arg et qui est absent ou cryptique dans le motif peptidique sialylé correspondant Cyc-_{D}Ala-Ser[Gal-Sial]-_{D}Ala-Arg ;
(b) la chaîne lourde de la molécule de liaison a la SEQ ID NO : 7 et/ou la chaîne légère de la molécule de liaison a la SEQ ID NO : 8 ; et/ou
(c) la molécule de liaison est marquée avec une molécule rapporteuse.

5. Dispositif ou kit selon l'une quelconque des revendications 1 à 4 dans lequel le site de capture de la molécule indicatrice :
(a) comprend ou se compose d'une molécule de biotine ou d'un fragment oxime ; et/ou se trouve à l'extrémité N ou C-terminale du peptide ; et/ou
(b) est lié au peptide par un lieur.

6. Dispositif ou kit selon l'une quelconque des revendications 1 à 5 dans lequel la molécule indicatrice :
(a) comprend ou se compose de la structure suivante :
(i) Cyc-_{D}Ala-Ser[Gal-Sial]-_{D}Ala-Arg-PEG-biotine
(ii) biotine-PEG-Asp-Glu-_{D}Ser-Nva-Cyc-_{D}Ala-Ser[Gal-Sial]-_{D}Ala-Arg-Phe-_{D}Ser-Val
(iii) biotine-PEG-Asp-Arg-_{D}Ala-BIP-Ser-Pro-Ser[Gal-Sial]-_{D}Ala-_{D}Asp-Ser
(iv) biotine-PEG-Asp-Ser-SEP(PO₃)-_{D}Ala-Ile-Orn-Ser[Gal-Sial]-_{D}Ala-Nle-Glu
(v) biotine-PEG-Asp-_{D}Ala-Arg-Nva-_{D}Ser-βAla-_{D}Ala-Nle-Ser[Gal-Sial]-BIP-Orn-_{D}Ala-Glu-Ser ; ou
(vi) biotine-PEG-Asp-Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val ; et/ou
(b) comprend (i) un peptide comprenant ou se composant de la séquence Cyc-_{D}Ala-Ser[Gal-Sial]-_{D}Ala-Arg ; et (ii) un site de capture qui comprend ou se compose d'une molécule biotine ou d'une fraction oxime.

7. Anticorps capable de se lier spécifiquement au (i) dérivé désialylé d'un peptide tel que défini dans l'une quelconque des revendications 1 à 3 ; ou à (ii) une molécule indicatrice tel que définie dans l'une quelconque des revendications 1 à 3 ou 5 à 6, dans lequel l'anticorps se lie de préférence au dérivé désialylé par rapport au peptide sialylé ou à la molécule indicatrice, et dans lequel l'anticorps a une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6.

8. Molécule indicatrice appropriée pour une utilisation dans la détection de la présente dans l'échantillon à tester d'une activité de clivage par une enzyme sialidase, la molécule indicatrice comprenant :
a) un peptide tel que défini dans l'une quelconque des revendications 1 à 3 ; et
b) un site de capture qui reste intact après le clivage du groupe sialyle de la molécule indicatrice par une enzyme sialidase présente dans l'échantillon, dans lequel la molécule indicatrice est de préférence telle que définie dans l'une quelconque des revendications 1 à 3 ou 5 à 6.

9. Peptide comprenant ou se composant de la séquence suivante :
(i) Cyc-_{D}Ala-Ser[Gal-Sial]-_{D}Ala-Arg (SEQ ID NO : 11)
(ii) Glu-_{D}Ser-Nva-Cyc-_{D}Ala-Ser[Gal-Sial]-_{D}Ala-Arg-Phe-_{D}Ser-Val (SEQ ID NO : 12)
(iii) Arg-_{D}Ala-BIP-Ser-Pro-Ser[Gal-Sial]-_{D}Ala-_{D}Asp-Ser (SEQ ID NO : 13)
(iv) Ser-SEP-_{D}Ala-Ile-Orn-Ser[Gal-Sial]-_{D}Ala-Nle-Glu (SEQ ID NO : 14)
(v) _{D}Ala-Arg-Nva-_{D}Ser-βAla-_{D}Ala-Nle-Ser[Gal-Sial]-BIP-Orn-_{D}Ala-Glu-Ser (SEQ ID NO : 15) ; ou
(vi) Thr-_{D}Ala-Nle-Glu-_{D}Ala-Arg-βAla-Cyc-Orn-_{D}Ser-Pro-Ser[Gal-Sial]-βAla-Nva-_{D}Ser-Glu-Thr-Cha-_{D}Ser-Val (SEQ ID NO : 16).

10. Peptide pour une utilisation dans la génération d'un anticorps selon la revendication 7, dans lequel le peptide est un dérivé désialylé du tel que défini dans l'une quelconque des revendications 1 à 3.

11. Procédé permettant de détecter la présence ou l'absence dans un échantillon à tester d'une activité de clivage d'une enzyme sialidase, le procédé comprenant :
(i) la mise en contact d'une molécule indicatrice telle que définie dans l'une quelconque des revendications 1 à 3 ou 5 à 6 avec l'échantillon à tester ;
(ii) l'ajout à l'échantillon à tester de molécules de liaison capables de se lier au dérivé désialylé de la molécule indicatrice, dans lequel les molécules de liaison sont incapables de se lier à la molécule indicatrice jusqu'à ce que le clivage du groupe sialyle de la molécule indicatrice par une enzyme sialidase présente dans l'échantillon se soit produit et dans lequel les molécules de liaison sont des anticorps ayant une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6 ;
(iii) la capture du dérivé désialylé de la molécule indicatrice au niveau d'une zone de capture par liaison de molécules de capture dans la zone de capture au site de capture, lesdites molécules de capture étant capables de se lier au site de capture indépendamment du fait que la molécule indicatrice a été clivée ou non ; et
(iv) la détection du clivage du groupe sialyle de la molécule indicatrice en déterminant la liaison des molécules de liaison au dérivé désialylé de la molécule indicatrice capturé dans la zone de capture.

12. Procédé permettant de détecter la présence ou l'absence dans un échantillon à tester d'une activité de clivage d'une enzyme sialidase, le procédé comprenant :
(i) l'ajout de l'échantillon à tester à une zone de capture comprenant des molécules de capture, lesdites molécules de capture étant liées au site de capture d'une molécule indicatrice telle que définie dans l'une quelconque des revendications 1 à 3 ou 5 à 6, dans lequel lesdites molécules de capture restent liées au site de capture indépendamment du fait qu'un clivage du groupe sialyle de la molécule indicatrice par l'enzyme sialidase présente dans l'échantillon se produise ou non ;
(ii) l'ajout de molécules de liaison capables de se lier au dérivé désialylé de la molécule indicatrice, dans lequel les molécules de liaison sont incapables de se lier à la molécule indicatrice jusqu'à ce que le clivage du groupe sialyle de la molécule indicatrice par l'enzyme sialidase présente dans le l'échantillon se soit produit et dans lequel les molécules de liaison sont des anticorps ayant une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6 ;
(iii) la détection du clivage du groupe sialyle de la molécule indicatrice en déterminant la liaison des molécules de liaison au dérivé désialylé de la molécule indicatrice capturé dans la zone de capture.

13. Procédé permettant de diagnostiquer une vaginose bactérienne dans un échantillon à tester en détectant l'activité de clivage d'une enzyme sialidase dans l'échantillon, le procédé comprenant :
(i) la mise en contact d'une molécule indicatrice telle que définie dans l'une quelconque des revendications 1 à 3 ou 5 à 6 avec l'échantillon à tester ;
(ii) l'ajout à l'échantillon à tester de molécules de liaison capables de se lier au dérivé désialylé de la molécule indicatrice, dans lequel les molécules de liaison sont incapables de se lier à la molécule indicatrice jusqu'à ce que le clivage du groupe sialyle de la molécule indicatrice par une enzyme sialidase présente dans l'échantillon se soit produit et dans lequel les molécules de liaison sont des anticorps ayant une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6 ;
(iii) la capture du dérivé désialylé de la molécule indicatrice au niveau d'une zone de capture par liaison de molécules de capture dans la zone de capture au site de capture, lesdites molécules de capture étant capables de se lier au site de capture indépendamment du fait que la molécule indicatrice a été clivée ou non ; et
(iv) la détection du clivage du groupe sialyle de la molécule indicatrice en déterminant la liaison des molécules de liaison au dérivé désialylé de la molécule indicatrice capturé dans la zone de capture, dans lequel un niveau de clivage accru par rapport à un témoin diagnostique une vaginose bactérienne.

14. Kit de détection d'enzymes permettant de détection la présence dans un échantillon à tester d'une activité de clivage d'une enzyme sialidase, le kit comprenant :
(i) une molécule indicatrice telle que définie dans l'une quelconque des revendications 1 à 3, dans lequel la molécule indicatrice est de préférence lyophilisée sur un support ;
(ii) des molécules de capture capables de se lier au site de capture de la molécule indicatrice, indépendamment du fait que la molécule indicatrice a été clivée ou non, dans lequel les molécules de capture comprennent de préférence de la streptavidine ;
(iii) un support solide auquel les molécules de capture peuvent être attachées, ou sont attachées, pour former une zone de capture pour recevoir l'échantillon à tester, dans lequel le support solide comprend de préférence de la nitrocellulose ; et
(iv) des molécules de liaison capables de se lier au dérivé désialylé de la molécule indicatrice, dans lequel l'anticorps se lie de préférence au dérivé désialylé par rapport au peptide sialylé ou à la molécule indicatrice, et dans lequel l'anticorps a une chaîne lourde avec 3 CDR et une chaîne légère avec 3 CDR, dans lequel la chaîne lourde CDR1 a la SEQ ID NO : 1 ; la chaîne lourde CDR2 a la SEQ ID NO : 2 ; la chaîne lourde CDR3 a la SEQ ID NO:3 ; la chaîne légère CDR1 a la SEQ ID NO : 4 ; la chaîne légère CDR2 a la SEQ ID NO : 5 ; et la chaîne légère CDR3 a la SEQ ID NO : 6.

15. Utilisation d'un dispositif de détection d'enzymes ou d'un kit de détection d'enzymes selon l'une quelconque des revendications 1 à 6, d'un procédé selon l'une quelconque des revendications 11 à 13 ou du kit de détection d'enzymes selon la revendication 14 permettant de diagnostiquer une vaginose bactérienne dans un échantillon à test.
